# EUROPEAN PATENT SPECIFICATION

(11) **EP 3 393 603 B1**
(45) Date of publication and mention of the grant of the patent: **18.01.2023**
(21) Application number: 16774717.9
(22) Date of filing: 26.08.2016
(51) Int. Cl.: A61Q 19/00, A61Q 19/02, A61Q 19/08, A61K 8/06, A61K 8/34, A61K 8/49, A61K 8/37, A61K 8/39, A61K 8/86, A61K 8/44

(54) **TOPICAL DELIVERY SYSTEM FOR ACTIVE INGREDIENTS**
TOPISCHES VERABREICHUNGSSYSTEM FÜR WIRKSTOFFE
SYSTÈME D'ADMINISTRATION PAR VOIE TOPIQUE DE PRINCIPES ACTIFS

(30) Priority: 22.12.2015 JP 2015250448
(43) Date of publication of application: 31.10.2018
(73) Proprietor: L'Oréal, 75008 Paris (FR)
(72) Inventor: ALAM, Mohammad Mydul, Kawasaki-shi Kanagawa 213-0012 (JP); IIMA, Yusuke, Kawasaki-shi Kanagawa 213-0012 (JP); KOIDE, Maki, Kawasaki-shi Kanagawa 213-0012 (JP); SINHA, Ritesh, Kawasaki-shi Kanagawa 213-0012 (JP)
(74) Representative: Casalonga
(86) International application number: PCT/JP2016/075740
(87) International publication number: WO 2017/110151

(56) References cited:
- EP-A1- 1 616 552
- WO-A1-2015/152420
- WO-A1-2015/198922
- CN-A- 101 401 775
- US-A1- 2012 328 702
- US-A1- 2015 335 560
- DATABASE WPI Week 201437 15 September 2014 (2014-09-15) Thomson Scientific, London, GB; AN 2014-K11357 XP002763472, & CN 103 690 380 A (PROYA COSMETICS CO LTD) 2 April 2014 (2014-04-02)
- DATABASE WPI Week 200962 10 September 2009 (2009-09-10) Thomson Scientific, London, GB; AN 2009-H44190 XP002763473, & CN 101 401 775 A (DING K) 8 April 2009 (2009-04-08)
- DATABASE GNPD [Online] MINTEL; 1 June 2013 (2013-06-01), Zydus Wellness: "Diamond Peptide Whitening Cream SPF 15", XP002763474, Database accession no. 2091271
- MASON T G ET AL: "TOPICAL REVIEW; Nanoemulsions: formation, structure, and physical properties", JOURNAL OF PHYSICS: CONDENSED MATTER, INSTITUTE OF PHYSICS PUBLISHING, BRISTOL, GB, vol. 18, no. 41, 18 October 2006 (2006-10-18), pages R635-R666, XP020102622, ISSN: 0953-8984, DOI: 10.1088/0953-8984/18/41/R01
- Nn: "Sodium (3-hydroxy-2-pentylcyclopentyl)acetate", , 21 August 2020 (2020-08-21), pages 1-1, XP055724319, Retrieved from the Internet: URL:https://echa.europa.eu/registration-do ssier/-/registered-dossier/19274/4/8 [retrieved on 2020-08-21]
- Nn: "Sodium (3-hydroxy-2-pentylcyclopentyl)acetate water solubility", , 21 August 2020 (2020-08-21), pages 1-1, XP055724322, Retrieved from the Internet: URL:https://echa.europa.eu/registration-do ssier/-/registered-dossier/19274/4/9 [retrieved on 2020-08-21]
- DATABASE GNPD [Online] MINTEL; 27 March 2015 (2015-03-27), anonymous: "Super Serum Correcting Perfect Skin", XP055724274, retrieved from www.gnpd.com Database accession no. 3082627
- DATABASE GNPD [Online] MINTEL; 19 November 2014 (2014-11-19), anonymous: "LR 2412 4% - Cx Advanced Skin Corrector", XP055724300, retrieved from www.gnpd.com Database accession no. 2807273
- Nn: "resorcinol; 1,3-benzenediol Substance Description Scientiic properties", , 30 July 2020 (2020-07-30), pages 1-4, XP055724329, Retrieved from the Internet: URL:https://echa.europa.eu/brief-profile/- /briefprofile/100.003.260 [retrieved on 2020-08-21]

## Description

### TECHNICAL FIELD

The present invention relates to a cosmetic composition, in particular a cosmetic composition in the form of an oil-in-water (O/W) nano-emulsion, for a keratin substance such as skin.

### BACKGROUND ART

The composition in a form of oil-in-water (O/W) emulsion is commonly used in a cosmetics and dermatological fields. To date, some prior art documents relating to cosmetic O/W emulsion compositions comprising cosmetic active ingredients, such as skin whitening and anti-aging agents, have been published.

JP-A-2011-73992 discloses a composition comprising the following components (A) to (F): (A) 10-50 parts by weight of one or more polyglycerin fatty acid esters with an HLB of 10-18, obtained from a polyglycerin and a fatty acid having 8 to 22 carbon atoms, (B) 1-30 parts by weight of one or more fatty acid monoglycerides obtained from glycerin and a fatty acid having 8 to 22 carbon atoms, (C) 0.1-30 parts by weight of an oil in the form of liquid at 25°C, (D) 10-35 parts by weight of a polyhydric alcohol, (E) 5-40 parts by weight of water, and (F) 0.01-10 parts by weight of a ceramide.

WO 2005/065630 A1 discloses a monophase microemulsion composition comprising (A) a hydrophilic nonionic surfactant, (B) a lipophilic nonionic surfactant, (C) oil, (D) an aqueous solvent immiscible with the oil, in which the critical micell concentration (c.m.c) of hydrophilic nonionic surfactant is higher than that in water, and (E) water.

WO 2004/045566 discloses a semitransparent cosmetic consisting of an O/W emulsion which comprises (a) a ceramide, (b) an oil component, (c) a nonionic surfactant, and (d) water and has a mean particle diameter of 100 to 300 nm.

CN101401775A discloses nanoemulsion with skin-whitening effect and preparation method thereof. When various types or skin-whitening functional components or additives are added, its particle size is about 100nm.

For cosmetic compositions including cosmetic active ingredients, such as skin whitening and anti-aging agents, penetration of the active ingredients through the skin is one of the most important properties. Therefore, there is a need to improve the penetration property of the active ingredients in cosmetic O/W emulsion compositions with a transparent aspect.

### DISCLOSURE OF INVENTION

An objective of the present invention is to provide an O/W composition, preferably a cosmetic O/W composition for a keratin substance, such as skin, which has an improved penetration property of the cosmetic active ingredients.

The above objective of the present invention can be achieved by a composition in the form of an O/W emulsion having an oil phase dispersed in an aqueous phase, said composition comprising:
(a) at least one oil;
(b) at least one active ingredient as defined below;
(c) water; and
(d) at least one surfactant selected from nonionic surfactants;
wherein:
- the diameter of the oil droplets of the oil phase is less than 30 nm, and
- the aqueous phase comprises less than 5 % by weight of water soluble alcohol, relative to the total amount of the aqueous phase,
   the water soluble alcohol being an alcohol which can dissolve in an amount of 1 g or more in 100 mL water at room temperature and under atmospheric pressure,
- the oil phase includes the active ingredient,
- the amount of the active ingredient in the oil phase is ranging from 0.1 to 20% by weight, relative to the total weight of the oil phase, and
- the amount of the water in the composition is from 60 to 99% by weight relative to the total weight of the composition.

The aqueous phase preferably comprises less than 1% by weight of water soluble alcohol, relative to the total amount of the aqueous phase, and more preferably the aqueous phase comprises no water soluble alcohol.

The diameter of the oil phase can be more than 1 nm, preferably more than 2 nm, and more preferably more than 5 nm.

The (b) active ingredient may preferably have a solubility in the oil ranging from 0.01 to 50% by weight, preferably from 0.05 to 40% by weight, and more preferably from 0.1 to 30% by weight.

The (b) active ingredient is selected from a group consisting of resorcinol derivatives as defined below, cinnamaldehyde derivatives as defined below, and a combination thereof.

The amount of the active ingredient in the oil phase ranges preferably from 1 to 15% by weight, and more preferably from 3 to 10% by weight, relative to the total weight of the oil phase.

The amount of the surfactant may be from 0.1 to 20% by weight, preferably from 0.5 to 15% by weight, more preferably from 1 to 10% by weight, relative to the total weight of the composition.

The amount of the oil may be from 0.1 to 15% by weight, preferably from 0.5 to 10% by weight, and more preferably from 1 to 5% by weight, relative to the total weight of the composition.

The amount of the water in the composition is preferably from 70 to 98% by weight, and more preferably 80 to 97% by weight, relative to the total weight of the composition.

Preferably, the oil is selected from a group consisting of ester oils having a molecular weight less than 600 g/mol such as isopropyl myristate, isopropyl palmitate, ethyl hexyl palmitate, isopropyl lauroyl sarcosinate, artificial triglycerides such as capryl caprylic triglycerides; hydrocarbon oil; and mineral oil such as paraffine oil, and mixtures thereof.

The surfactant may be selected from a group consisting of polyoxyethylene alkyl ether carboxylic acids, such as Laureth-5 Carboxylic Acid, ethers of a sugar and of C₈-C₂₄ fatty alcohols, such as caprylyl/capryl glucoside, polyoxyethylenated fatty alcohol containing from 6 to 12 oxyethylene units, such as Laureth-9, polyoxyalkylenated derivative of mono glyceryl ester of a fatty acid such as PEG-20 glyceryl triisostearate, polyglyceryl esters of a fatty acid, such as polyglyceryl-2 oleate, sarcosinates, such as sodium lauroyl sarcosinate, and mixtures thereof.

Preferably, the appearance of the composition is transparent or translucent.

The present invention also relates to a cosmetic process for a keratin substance such as skin, comprising the step of: applying onto the keratin substance the composition according to the present invention.

### BEST MODE FOR CARRYING OUT THE INVENTION

After diligent research, the inventors have found that an elimination of water soluble alcohol from an O/W emulsion composition can surprisingly improve a penetration property of an active ingredient through a keratin substance, and thus improve its bioavailability.

Thus, the composition, preferably a cosmetic composition for keratin substance, preferably skin, according to the present invention is in the form of an O/W emulsion having an oil phase dispersed in an aqueous phase and said composition comprises:
(a) at least one oil;
(b) at least one active ingredient as defined below;
(c) water; and
(d) at least one surfactant selected from nonionic surfactants;
wherein
- the diameter of the oil droplets of the oil phase is less than 30 nm, and
- the aqueous phase comprises less than 5 % by weight of water soluble alcohol, relative to the total amount of the aqueous phase,
   the water soluble alcohol being an alcohol which can dissolve in an amount of 1 g or more in 100 mL water at room temperature and under atmospheric pressure,
- the oil phase includes the active ingredient,
- the amount of the active ingredient in the oil phase is ranging from 0.1 to 20% by weight, relative to the total weight of the oil phase, and
- the amount of the water in the composition is from 60 to 99% by weight relative to the total weight of the composition.

The aqueous phase preferably comprises less than 1% by weight of water soluble alcohol, relative to the total amount of the aqueous phase and more preferably the aqueous phase comprises no water soluble alcohol.

The composition according to the present invention can exhibit an improved penetration property of the active ingredient in the oil phase through a keratin substance.

Hereafter, the composition according to the present invention will be described in a detailed manner.

### [Composition]

The composition according to the present invention is in the form of an O/W emulsion having oil phases dispersed in an aqueous phase. The composition is as defined in claim 1 and comprises (a) at least one oil, (b) at least one active ingredient as defined below, (c) water, and (d) at least one surfactant selected from nonionic surfactants.

It is preferable that the composition according to the present invention is a cosmetic composition, in particular a cosmetic composition for a keratin substance such as skin, i.e. a skin care cosmetic composition.

The composition according to the present invention can exhibit an improved penetration property of the active ingredients thorough a keratinous substance, preferably skin. Furthermore, the composition according to the present invention can have a transparent or translucent appearance, which is preferable in use for cosmetic topical compositions for a keratin substance, such as skin.

### Oil Phase

The oil phase of the O/W emulsion according to the present invention includes at least one (a) oil. The oil phase is preferably in the form of a nano-sized droplet. The diameter of the oil phase, i.e. oil droplet, is less than 30 nm. In general, the diameter of the oil phase is preferably more than 1 nm, more preferably more than 2 nm, and even more preferably more than 5 nm. The size of the oil droplet can be measured by, for example, Particle size analyzer (Vasco, Cordouan Technologies). The diameter of the oil phase means number mean diameter. A nano-sized oil phase may be advantageous in that the active ingredient in the oil phase can be applied on a keratinous substance homogeneously and thickly, and thus it also may improve bioavailability of the active ingredients.

### (Oil)

The composition according to the present invention comprises (a) at least one oil. Two or more (a) oils may be used in combination. Thus, a single type of oil or a combination of different types of oil may be used.

As used herein, the expression "oil" means a fatty compound or substance which is in the form of a liquid or a paste (non-solid) at room temperature (25°C) under atmospheric pressure (760 mmHg). These oil(s) may be volatile or non-volatile, preferably non-volatile.

The (a) oil may be a non-polar oil such as a hydrocarbon oil, a silicone oil, or the like; a polar oil such as a plant or animal oil and an ester oil or an ether oil; or a mixture thereof.

It is preferable that the (a) oil be selected from the group consisting of oils of plant or animal origin, synthetic oils, silicone oils, and hydrocarbon oils, and mixtures thereof.

As examples of plant oils, mention may be made of, for example, linseed oil, camellia oil, macadamia nut oil, corn oil, mink oil, olive oil, avocado oil, sasanqua oil, castor oil, safflower oil, jojoba oil, sunflower oil, almond oil, rapeseed oil, sesame oil, soybean oil, peanut oil, and mixtures thereof.

As examples of animal oils, mention may be made of, for example, squalene and squalane.

As examples of synthetic oils, mention may be made of alkane oils such as isododecane and isohexadecane, ester oils, ether oils, and artificial triglycerides.

The ester oils are preferably liquid esters of saturated or unsaturated, linear or branched C₁-C₂₆ aliphatic monoacids or polyacids and of saturated or unsaturated, linear or branched C₁-C₂₆ aliphatic monoalcohols or polyalcohols, the total number of carbon atoms of the esters being greater than or equal to 10.

Preferably, for the esters of monoalcohols, at least one from among the alcohol and the acid from which the esters of the invention are derived is branched.

Among the monoesters of monoacids and of monoalcohols, mention may be made of ethyl palmitate, ethyl hexyl palmitate, isopropyl palmitate, dicaprylyl carbonate, alkyl myristates such as isopropyl myristate or ethyl myristate, isocetyl stearate, 2-ethylhexyl isononanoate, isononyl isononanoate, isodecyl neopentanoate and isostearyl neopentanoate.

Esters of C₄-C₂₂ dicarboxylic or tricarboxylic acids and of C₁-C₂₂ alcohols and esters of monocarboxylic, dicarboxylic or tricarboxylic acids and of non-sugar C₄-C₂₆ dihydroxy, trihydroxy, tetrahydroxy or pentahydroxy alcohols may also be used.

Mention may especially be made of: diethyl sebacate; isopropyl lauroyl sarcosinate; diisopropyl sebacate; bis(2-ethylhexyl) sebacate; diisopropyl adipate; di-n-propyl adipate; dioctyl adipate; bis(2-ethylhexyl) adipate; diisostearyl adipate; bis(2-ethylhexyl) maleate; triisopropyl citrate; triisocetyl citrate; triisostearyl citrate; glyceryl trilactate; glyceryl trioctanoate; trioctyldodecyl citrate; trioleyl citrate; neopentyl glycol diheptanoate; diethylene glycol diisononanoate.

As ester oils, one can use sugar esters and diesters of C₆-C₃₀ and preferably C₁₂-C₂₂ fatty acids. It is recalled that the term "sugar" means oxygen-bearing hydrocarbon-based compounds containing several alcohol functions, with or without aldehyde or ketone functions, and which comprise at least 4 carbon atoms. These sugars may be monosaccharides, oligosaccharides or polysaccharides.

Examples of suitable sugars that may be mentioned include sucrose (or saccharose), glucose, galactose, ribose, fucose, maltose, fructose, mannose, arabinose, xylose and lactose, and derivatives thereof, especially alkyl derivatives, such as methyl derivatives, for instance methylglucose.

The sugar esters of fatty acids may be chosen especially from the group comprising the esters or mixtures of esters of sugars described previously and of linear or branched, saturated or unsaturated C₆-C₃₀ and preferably C₁₂-C₂₂ fatty acids. If they are unsaturated, these compounds may have one to three conjugated or non-conjugated carbon-carbon double bonds.

The esters according to this variant may also be selected from monoesters, diesters, triesters, tetraesters and polyesters, and mixtures thereof.

These esters may be, for example, oleates, laurates, palmitates, myristates, behenates, cocoates, stearates, linoleates, linolenates, caprates and arachidonates, or mixtures thereof such as, especially, oleopalmitate, oleostearate and palmitostearate mixed esters, as well as pentaerythrityl tetraethyl hexanoate.

More particularly, use is made of monoesters and diesters and especially sucrose, glucose or methylglucose monooleates or dioleates, stearates, behenates, oleopalmitates, linoleates, linolenates and oleostearates.

An example that may be mentioned is the product sold under the name Glucate^{®} DO by the company Amerchol, which is a methylglucose dioleate.

As examples of preferable ester oils, mention may be made of, for example, diisopropyl adipate, dioctyl adipate, 2-ethylhexyl hexanoate, ethyl laurate, cetyl octanoate, octyldodecyl octanoate, isodecyl neopentanoate, myristyl propionate, 2-ethylhexyl 2-ethylhexanoate, 2-ethylhexyl octanoate, 2-ethylhexyl caprylate/caprate, methyl palmitate, ethyl palmitate, isopropyl palmitate, dicaprylyl carbonate, isopropyl lauroyl sarcosinate, isononyl isononanoate, ethylhexyl palmitate, isohexyl laurate, hexyl laurate, isocetyl stearate, isopropyl isostearate, isopropyl myristate, isodecyl oleate, glyceryl tri(2-ethylhexanoate), pentaerythrithyl tetra(2-ethylhexanoate), 2-ethylhexyl succinate, diethyl sebacate, and mixtures thereof.

As examples of artificial triglycerides, mention may be made of, for example, capryl caprylic triglycerides, glyceryl trimyristate, glyceryl tripalmitate, glyceryl trilinolenate, glyceryl trilaurate, glyceryl tricaprate, glyceryl tricaprylate, glyceryl tri(caprate/caprylate) and glyceryl tri(caprate/caprylate/linolenate).

As examples of silicone oils, mention may be made of, for example, linear organopolysiloxanes such as dimethylpolysiloxane, methylphenylpolysiloxane, methylhydrogenpolysiloxane, and the like; cyclic organopolysiloxanes such as octamethylcyclotetrasiloxane, decamethylcyclopentasiloxane, dodecamethylcyclohexasiloxane, and the like; and mixtures thereof.

Preferably, the oil phase of the present invention does not include any silicone oil. In other words, the (a) oil of the present invention may be selected from non-silicone oil.

Hydrocarbon oils may be chosen from:
- linear or branched, optionally cyclic, C₆-C₁₆ lower alkanes. Examples that may be mentioned include hexane, undecane, dodecane, tridecane, and isoparaffins, for instance isohexadecane, isododecane and isodecane; and
- linear or branched hydrocarbons containing more than 16 carbon atoms, such as liquid paraffins, liquid petroleum jelly, polydecenes and hydrogenated polyisobutenes such as Parleam^{®}, and squalane.

As preferable examples of hydrocarbon oils, mention may be made of, for example, linear or branched hydrocarbons such as isohexadecane, isododecane, squalane, mineral oil (e.g., liquid paraffin), paraffin, vaseline or petrolatum, naphthalenes, and the like; hydrogenated polyisobutene, isoeicosane, and decene/butene copolymer; and mixtures thereof.

It may be preferable that the (a) oil is chosen from non-polar hydrocarbon oils which are in the form of a liquid at a room temperature.

It may be also preferable that the (a) oil be chosen from polar oils with molecular weight below 600 g/mol.

Preferably, the (a) oil has a low molecular weight such as below 600 g/mol, more preferably below 500 g/mol, in particular below 400 g/mol, chosen among ester or ether oils with a short hydrocarbon chain or chains (C₁-C_{18,} e.g., isopropyl myristate, isopropyl palmitate, isononyl isononanoate, dicaprylyl carbonate, ethyl hexyl palmitate, dicaprylyl ether, and isopropyl lauroyl sarcosinate, artificial triglycerides such as capryl caprylic triglycerides), hydrocarbon oils with a short alkyl chain or chains (C₁-C₁₈, e.g., isododecane, isohexadecane, and squalane), and short alcohol type oils such as octyldodecanol.

It is also preferable that the (a) oil be selected from the group consisting of hydrocarbon oils, esters of C₄-C₂₂ dicarboxylic or tricarboxylic acids and of C₁-C₂₂ alcohols, and esters of C4-C22 monocarboxylic, dicarboxylic or tricarboxylic acids and of non-sugar C₄-C₂₆ dihydroxy, C₄-C₁₅ trihydroxy, tetrahydroxy or pentahydroxy alcohols, and mixtures thereof.

Preferably, the (a) oil is selected from a group consisting of ester oils having a molecular weight less than 600 g/mol such as isopropyl myristate, isopropyl palmitate, ethyl hexyl palmitate and isopropyl lauroyl sarcosinate, artificial triglycerides such as capryl caprylic triglycerides; hydrocarbon oil; and mineral oil such as paraffine oil, and mixtures thereof.

The amount of the (a) oil in the composition according to the present invention is not limited, and may range from 0.01 to 20% by weight, preferably from 0.1 to 15% by weight, more preferably from 0.5 to 10% by weight, in particular 1 to 5 % by weight, relative to the total weight of the composition.

### (Active Ingredient)

The composition according to the present invention comprises (b) at least one active ingredient in the oil phase. Therefore, the active ingredient is preferably oil soluble and is relatively poorly-water soluble. Two or more (b) active ingredients may be used in combination. Thus, a single type of an active ingredient or a combination of different types of active ingredients may be used.

The term "active ingredient" used herein means an organic compound having any cosmetic or dermatological effects on a keratinous substance, such as skin. In particular, the active ingredients preferably exhibit cosmetic or dermatological effects on a keratinous substance, such as skin, after they penetrate the keratinous substance.

The active ingredient is selected from resorcinol derivatives represented by formula (I), cinnamaldehyde derivatives selected from trans-ferulic acid, p-coumaric acid, and coniferylaldehyde, and a combination thereof. Said active ingredients are skin-whitening ingredients.

The resorcinol derivative used in the present invention is a compound represented by the formula (I): 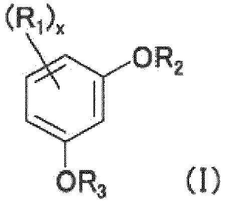 wherein
R₁ independently denotes -A-B where A represents a single bond, a C₁-C₆ alkylene group, a C₆-₁₂ arylene group, or a C₁₋₆ alkylene-C₆₋₁₂ arylene group, and B represents a halogen atom, -OH, -COH, -COOH, -CONH₂, -NH₂, a C₁-C₆ alkyl group, a C₁₋₆ alkoxy group, a C₁₋₆ acyl group, a carbocyclic group, preferably an aryl group, or heterocyclic group, preferably a non-aromatic heterocyclic group, each of which may be substituted with at least one substituent selected from the group consisting of a hydroxyl group, a carboxyl group, a C₁₋₆ alkyl group, a C₁₋₆ alkylene-OH, an amino group, -CONH₂, -CONH-C₁₋₆ alkyl group and a C₁₋₆ alkoxy group;
x is an integer of 1 to 4, preferably 1 to 3, more preferably 1 or 2 and even more preferably 1; and
R₂ and R₃ independently denote a hydrogen atom, a C₁₋₆ alkyl group, or a C₁₋₆ acyl group,
or a salt thereof.

The C₁-C₆ alkylene group may be a straight or branched divalent group.

The C₁₋₆ alkylene-C₆₋₁₂ arylene group may also be a straight or branched divalent group. Either the C₁₋₆ alkylene moiety or the C₆-₁₂ arylene moiety may bond the dihydroxy benzene ring shown in the formula (I).

The aryl group as "B" may be a C₆-₁₂ aryl group such as a phenyl group, a tolyl group and a xylyl group, or a naphtyl group.

The hetero atom in the heterocyclic group as "B" may be an oxygen atom, a sulfur atom and a nitrogen atom. A single heteroatom or a plurality of hetero atoms may be included in the heterocyclic group. As examples of the heterocyclic group, mention may be made of a furanyl group, a pyrrole group, an oxazole group, an isoxazolyl group, a thiazolyl group, an isothiazolyl group, an imidazolyl group, a pyrazolyl group, a pyranyl group, a pyridinyl group, a pyridazinyl group, a pyrimidinyl group, a pyrazinyl group, an indolyl group, an isoindolyl group, a benzofuranyl group, a quinolinyl group, isoquinolinyl group and an indazolyl group.

As examples of the non-aromatic heterocyclic group, mention may be made of a pyrrolidinyl group, an imidazolidinyl group, a pyrazolidinyl group, a piperidinyl group, a piperazinyl group, a morpholinyl group, and a tetrahydropyranyl group.

As examples of the compound according to formula (I), mention may be made of: 2-methylresorcinol, 5-methylresorcinol, 4-methylresorcinol, 2,4-dihydroxybenzaldehyde, 4-ethylresorcinol, 2,5-dimethylresorcinol, 4,5-dimethylresorcinol, 2,4-dimethyl-1,3-benzenediol, 3,5-dihydroxybenzylamine, 5-methoxyresorcinol, 3,5-dihydroxybenzyl alcohol, 2-methoxyresorcinol, 4-methoxyresorcinol, 3,5-dihydroxytoluene monohydrate, 4-chlororesorcinol, 2-chlororesorcinol, 2',4'-dihydroxyacetophenone, 3',5'-dihydroxyacetophenone, 2,6-dihydroxy-4-methylbenzaldehyde, 4-propylresorcinol, 2,4-dihydroxy-1,3,5-trimethylbenzene, 3,5-dihydroxybenzamide, 2,6-dihydroxybenzamide, 2,4-dihydroxybenzamide, 2,4-dihydroxybenzoic acid, 2,6-dihydroxybenzoic acid, 3,5-dihydroxybenzoic acid, 2,6-dihydroxy-4-methylbenzyl alcohol, 3,5-dihydroxyanisole hydrate, 4-aminoresorcinol hydrochloride, 2-aminoresorcinol hydrochloride, 5-aminobenzene-1,3-diol hydrochloride, 2',4'-dihydroxypropiophenone, 2',4'-dihydroxy-3'-methylacetophenone, (2,4-dihydroxyphenyl)acetone, (3,5-dihydroxyphenyl)acetone, 2,6-dihydroxy-4'-methylacetophenone, 4-n-butylresorcinol, 2,4-diethyl-1,3-benzenediol, 3,5-dihydroxy-4-methylbenzoic acid, 2,6-dihydroxy-4-methylbenzoic acid, 2,4-dihydroxy-6-methylbenzoic acid, 3,5-dihydroxyphenylacetic acid, 2-ethyl-5-methoxybenzene-1,3-diol, 4-amino-3,5-dihydroxybenzoic acid, 3,5-dihydroxyacetophenone monohydrate, 3,5-dihydroxybenzylamine hydrochloride, 4,6-dichlororesorcinol, 2',4'-dihydroxy-3'-methylpropiophenone, 1-(3-ethyl-2,6-dihydroxyphenyl)ethan-1-one, 2',6'-dihydroxy-4'-methoxyacetophenone, 1-(2,6-dihydroxy-3-methoxyphenyl)ethan-1-one, 3(2,4-dihydroxyphenylpropionic acid, and 2,4-dihydroxy-3,6-dimethylbenzoic acid.

In one embodiment, the resorcinol derivative may be a compound represented by the formula (Ia): 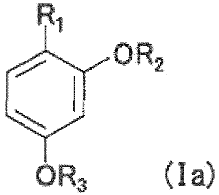 wherein
R₁, R₂ and R₃ have the same meaning as above,
or a salt thereof.

It is preferable that, in the above formula (Ia),
R¹ denote -A-B where A represents a single bond or a C₁₋₆ alkylene group, and B represents a phenyl group or a tetrahydropyranyl group; and
each of R₂ and R₃ denote a hydrogen atom.

It is even more preferable that the resorcinol derivative be phenylethyl resorcinol and 4-(tetrahydro-2H-pyran-4-yl)benzene-1,3-diol.

In one embodiment, the resorcinol derivative may be a compound represented by the formula (II): 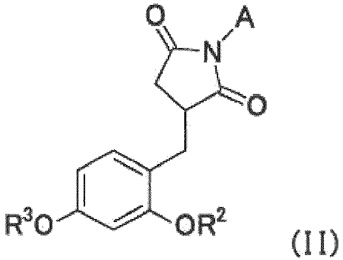 wherein
R₂ and R₃ independently denote a hydrogen atom or an acetyl group;
A denotes a radical selected from:
   a) -H;
   b) - a C₃-C₈ cyclic or C₃-C₂₀ branched or C₂-C₂₀ unsaturated or C₁-C₂₀ saturated linear alkyl group which is optionally interrupted by one or more heteroatoms or moieties selected from N, O and -CO- or a combination thereof such as -NHCO-, -NHCONH- and/or is optionally substituted by one or more identical or different groups selected from:
      i) -OR₅
      ii) -SR₅
      iii) -NR₆R₇
      iv) -CONHR₆
      v) -CONR₆R₇
      vi) -COOR₆
      vii) -NHCONHR6
      viii) -C(O)C₁-C₄ alkyl
      ix) a C₅-C₁₂ (hetero)aryl group optionally containing one or more heteroatoms selected from O, N and S and optionally substituted by one or more hydroxyls and/or by one or more C₁-C₈ alkoxy radicals;
      x) a saturated or unsaturated, non-aromatic heterocycle having from 5 to 8 members and comprising one or more heteroatoms selected from O, N and S which is optionally substituted by one or more hydroxyls and/or by one or more C₁-C₈ alkoxy or C₁-C₄ alkyl radicals, it being possible for one of the members to be a carbonyl group;
   c) - a C₅-C₁₂ (hetero)aryl group optionally containing one or more heteroatoms selected from O, N and S and optionally substituted by one or more hydroxyls and/or by one or more radicals selected from C₁-C₈ alkoxy or C₁-C₈ alkyl groups;
   d) -NR₈R₉;
   e) -OR₄;
   f) -C(O)NHR₄;
   g) C(O)C₁-C₁₀ alkyl,
      where
      R₈ and R₉, which are identical or different, denote a radical selected from:
         a) -H;
         b) - a C₃-C₈ cyclic or C₃-C₁₀ branched or C₂-C₁₀ unsaturated or C₁-C₁₀ linear saturated alkyl group which is optionally interrupted by one or more heteroatoms or moieties selected from N, O and -CO- or a combination thereof such as -NHCO-, -NHCONH- and/or is optionally substituted by one or more identical or different groups selected from -OR₅;
         c) a C₅-C₁₂ (hetero)aryl group optionally containing one or more heteroatoms selected from O, N and S and optionally substituted by one or more hydroxyls and/or by one or more C₁-C₈ alkoxy radicals;
      it being possible for R₈ and R₉ to form, with the nitrogen which carries them, a heterocycle which has from 5 to 8 members and may contain one or more heteroatoms or moieties selected from N, O and -CO- and/or is optionally substituted by a C₁-C₁₀ hydrocarbon chain optionally containing one or more radicals selected from hydroxyl or C₁-C₄ alkoxy;
      R₄ denotes a radical selected from:
         a) -H
         b) a C₃-C₈ cyclic or C₃-C₁₀ branched or C₁-C₁₀ linear saturated alkyl group which is optionally substituted by one or more identical or different groups selected from:
            i) -COOR₆,
            ii) a C₅-C₁₂ (hetero)aryl radical which optionally contains one or more heteroatoms selected from O, N and S and is optionally substituted by one or more hydroxyls and/or by one or more C₁-C₈ alkoxy radicals;
         c) a C₅-C₁₂ (hetero)aryl group which optionally contains one or more heteroatoms selected from O, N and S and is optionally substituted by one or more hydroxyls and/or by one or more C₁-C₈ alkoxy radicals;
      R₅ is selected from H and a C₃-C₈ cyclic or C₂-C₁₀ unsaturated or C₃-C₁₀ branched or C₁-C₁₀ linear saturated alkyl hydrocarbon group;
      R₆ and R₇, which are identical or different, are selected from H, a C₃-C₈ cyclic or C₂-C₁₀ unsaturated or C₃-C₁₀ branched or C₁-C₁₀ linear saturated alkyl hydrocarbon group; a (C₁-C₄)alkyl-C₆ (hetero)aryl group optionally containing a nitrogen atom, more particularly a benzyl group;
      R₆ and R₇ may form, with the nitrogen which carries them, a heterocycle which has from 5 to 8 members and may contain one or more heteroatoms or moieties selected from N, O and -CO- and/or is optionally substituted by a C₁-C₁₀ hydrocarbon chain;
   h) a radical of formula (III): 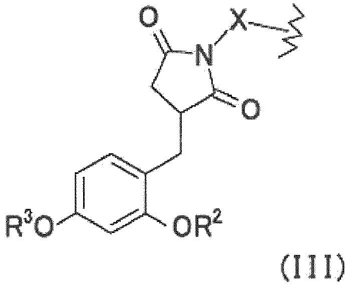 in which:
      X denotes a C₃-C₈ cyclic or C₃-C₁₀ branched or C₁-C₁₀ linear saturated hydrocarbon chain or a C₆-C₁₂ arylene group such as phenylene, or a C₁-C₄ alkylene-C₆-C₈ cycloalkylene-C₁-C₄ alkylene group or a C₁-C₄ alkylene-phenylene-C₁-C₄ alkylene group, which is optionally substituted by one or more identical or different radicals selected from -OH, -COOR₆ where
      R₆ denotes H or a C₃-C₈ cyclic or C₂-C₁₀ unsaturated or C₃-C₁₀ branched or C₁-C₂₀ linear saturated alkyl hydrocarbon group;
      R₂ and R₃ have the same meaning as above; and
      when A denotes a radical of formula (III), all of the radicals R₂ and R₃ in the compounds of formula (II) are identical,
      or a salt thereof.

The salts of the compounds of formulae (I) and (II) include conventional non-toxic salts of said compounds, such as those formed from an acid or from a base.

Salts of the compound of formulae (I) and (II) (when it comprises a quaternizable nitrogen atom) include the following:
a) salts obtained by addition of the compound (I) or (II) with a mineral acid, selected more particularly from hydrochloric, boric, hydrobromic, hydroic, sulphuric, nitric, carbonic, phosphoric and tetrafluoroboric acids;
b) or the salts obtained by addition of the compound (I) or (II) with an organic acid, more particularly selected from acetic, propionic, succinic, fumaric, lactic, glycolic, citric, gluconic, salicylic, tartaric, terephthalic, methylsulphonic, ethylsulphonic, benzene sulphonic, toluene sulphonic and triflic acids.

Also included are the salts obtained by addition of the compound of formula (I) or (II) (when it comprises an acidic group) with a mineral base, such as aqueous sodium hydroxide and potassium hydroxide, calcium hydroxide, ammonium hydroxide, magnesium hydroxide, lithium hydroxide, and sodium, potassium or calcium carbonates or hydrogencarbonates, for example; or with an organic base such as a primary, secondary or tertiary alkylamine, for example triethylamine or butylamine. This primary, secondary or tertiary alkylamine may comprise one or more nitrogen and/or oxygen atoms and may therefore comprise, for example, one or more alcohol functions; included more particularly are 2-amino-2-methylpropanol, ethanolamine, triethanolamine, 2-dimethylamino propanol, 2-amino-2-(hydroxymethyl)-1,3-propanediol and 3-(dimethylamino)propylamine.

Also included are the salts of amino acids such as, for example, lysine, arginine, guanidine, glutamic acid and aspartic acid.

The salts of the compounds of formulae (I) and (II) (when it comprises an acidic group) may advantageously be selected from alkali metal salts or alkaline earth metal salts such as sodium, potassium, calcium and magnesium salts; and ammonium salts.

The salts of the compounds of formulae (I) and (II) (when it comprises a quaternizable nitrogen atom) may advantageously be selected from halides such as chloride and bromide; and from citrates, acetates, succinates, phosphates, lactates and tartrates.

The linear or branched groups may preferably be selected from methyl, ethyl, propyl, isopropyl, butyl, isobutyl, tert-butyl, pentyl, hexyl, heptyl, octyl, nonyl, decyl, undecyl, dodecyl, tridecyl, tetradecyl, pentadecyl, hexadecyl, heptadecyl, octadecyl, nonadecyl and eicosyl.

The saturated linear or branched alkyl groups may more preferably be selected from methyl, ethyl, propyl, isopropyl, butyl, isobutyl, tert-butyl, pentyl, hexyl, heptyl and octyl.

The C₁-C₄ alkoxy groups may preferably be selected from methoxy, ethoxy, propoxy and butoxy and more preferably methoxy.

The compounds of formula (II) preferably have the following meanings:
R₂ and R₃ independently denote a hydrogen atom or an acetyl group;
A denotes a radical selected from:
   a) - H
   b) - a C₃-C₈ cyclic or C₃-C₁₆ branched or C₂-C₁₆ unsaturated or C₁-C₁₆ linear saturated alkyl group which is optionally interrupted by one or more heteroatoms or moieties selected from N, O, -CO- and -NHC(O)- and/or is optionally substituted by one or more identical or different groups selected from:
      i) -OH,
      ii) C₁-C₄ alkoxy,
      iii) -COOR₆,
      iv) -CONR₆R₇ where R₆ and R₇, which are identical or different, denote H or a C₃-C₈ cyclic or C₂-C₈ unsaturated or C₃-C₈ branched or C₁-C₈ linear saturated alkyl group;
      v) a phenyl group which is optionally substituted by one or more hydroxyls and/or by one or more C₁-C₄ alkoxy radicals;
      vi) a non-aromatic saturated or unsaturated heterocycle having from 5 to 8 members, comprising one or more heteroatoms selected from O, N and S, it being possible for one of the members to be a carbonyl group;
   c) a C₅-C₁₂ aryl group such as phenyl which is optionally substituted by one or more identical or different radicals selected from OH, C₁-C₄ alkoxy and C₁-C₄ alkyl;
   d) -NR₈R₉, where R₈ and R₉, which are identical or different, denote:
      i) H;
      ii) a C₃-C₈ cyclic or C₂-C₈ unsaturated or C₃-C₈ branched or C₁-C₈ linear saturated alkyl group which is optionally interrupted by an oxygen atom and/or is optionally substituted by a hydroxyl group or a C₁-C₄ alkoxy group such as methoxy;
      iii) a C₅-C₁₂ aryl group which is optionally substituted by one or more hydroxyls and/or by one or more C₁-C₄ alkoxy radicals;
      it being possible for R₈ and R₉ to form, with the nitrogen which carries them, a heterocycle having from 5 to 8 members, said heterocycle being able to contain one or more oxygen atoms and/or being optionally substituted by a C₁-C₆ hydrocarbon chain optionally containing one or more radicals selected from hydroxyl or C₁-C₄ alkoxy;
   e) -OR₄
   f) -C(O)NHR₄,
      where R₄ denotes a radical selected from -H, a C₃-C₈ branched or C₁-C₈ linear saturated alkyl group which is optionally substituted by one or more identical or different groups selected from:
      i) -COOR₆, where R₆ is as defined above;
      ii) a C₅-C₁₂ aryl radical,
   g) a radical of formula (III) 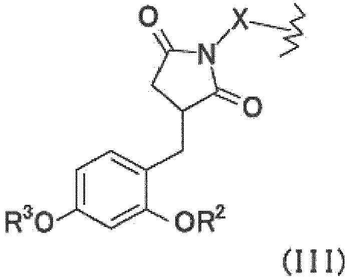 in which X denotes a C₃-C₈ cyclic or C₃-C₆ branched or C₁-C₆ linear saturated hydrocarbon chain or a C₆-C₁₂ arylene group such as phenylene, which is optionally substituted by one or more identical or different radicals selected from OH or a C₁-C₆ alkyl group,
      R₂ and R₃ have the same meaning as above;
      and
      when A denotes a radical of formula (III), all of the radicals R₂ and R₃ in the compounds of formula (II) are identical.

The compounds of formula (I) more preferably have the following meanings:
R₂ and R₃ independently denote a hydrogen atom or an acetyl group;
A denotes a radical selected from:
   a) H
   b) a C₃-C₈ cyclic or C₃-C₁₆ branched or C₂-C₁₆ unsaturated or C₁-C₁₆ linear saturated alkyl group which is optionally interrupted by one or more heteroatoms selected from N and O and/or is optionally substituted by one or more identical or different groups selected from:
      i) -OH
      ii) C₁-C₄ alkoxy,
      iii) -CONH₂;
      iv) -COOR₆, where R₆ denotes H or a C₃-C₄ cyclic or C₂-C₄ unsaturated or C₃-C₄ branched or C₁-C₄ linear saturated alkyl group;
      v) a phenyl group which is optionally substituted by one or more hydroxyls and/or by one or more C₁-C₄ alkoxy radicals;
      vi) a saturated or unsaturated, non-aromatic heterocycle having from 5 to 8 members, comprising one or more nitrogen atoms, it being possible for one of the members to be a carbonyl moiety;
   c) a C₅-C₁₂ aryl group such as phenyl;
   d) -NR₈R₉, where R₈ and R₉, which are identical or different, denote H or a C₃-C₈ cyclic or C₂-C₆ unsaturated or C₃-C₆ branched or C₁-C₆ linear saturated alkyl group; or a C₅-C₁₂ aryl group such as phenyl;
      it being possible for R₈ and R₉ to form, with the nitrogen which carries them, a heterocycle having from 5 to 8 members, it being possible for said heterocycle to contain an oxygen atom and/or being optionally substituted by a C₁-C₆ hydrocarbon chain optionally containing one or more radicals selected from hydroxyl or C₁-C₄ alkoxy;
   e) -OR₄, where R₄ denotes H or a C3-C6 branched or C₁-C₆ linear saturated alkyl group which is optionally substituted by one or more identical or different groups selected from:
      i) -COOH,
      ii) a C₅-C₁₂ aryl radical such as phenyl;
   f) a radical of formula (III) 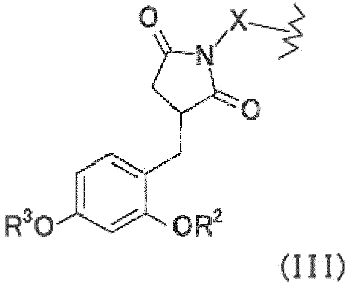 in which X denotes a C₃-C₈ cyclic or C₃-C₆ branched or C₁-C₆ linear saturated hydrocarbon chain or a C₆-C₁₂ arylene group such as phenylene, which is optionally substituted by one or more hydroxyl radicals;
      R₂ and R₃ have the same meaning as above;
      and
      when A denotes a radical of formula (III), all of the radicals R₂ and R₃ in the compounds of formula (II) are identical.

Preferentially, R₂ and R₃ = H for the compounds of formula (II).

A number of embodiments of compounds of formula (II) are described below:
R₂ and R₃ = H, and A = H.
R₂ and R₃ = H, and A = C₃-C₁₆ branched or C₁-C₁₆ saturated linear alkyl group.
R₂ and R₃ = H, and A = C₃-C₈ branched or C₁-C₈ saturated linear alkyl group which is substituted by one or two hydroxyl groups and is optionally substituted by a group -SR₅, where R₅ = H or C₁-C₄ alkyl.
R₂ and R₃ = H, and A = phenyl or benzyl group.
R₂ and R₃ = H, and A = C₃-C₈ branched or C₁-C₈ alkyl group which is substituted by a phenyl group which is optionally substituted by one or more hydroxyl groups and/or C₁-C₄ alkoxy group.
R₂ and R₃ = H, and A = C₃-C₈ branched or C₁-C₈ saturated linear alkyl group which is substituted by a -COOH group, which is optionally substituted by a group SR₅, where R₅ = H or C₁-C₄ alkyl.
R₂ and R₃ = H, and A = C₃-C₈ branched or C₁-C₈ saturated linear alkyl group which is substituted by a group -COOR₆, where R₆ denotes a C₁-C₆ alkyl group, and is optionally substituted by a hydroxyl group and/or a group -SR₅, where R₅ = H or C₁-C₄ alkyl and/or phenyl which is optionally substituted by one or more hydroxyls, or an imidazole radical.
R₂ and R₃ = H, and A = C₃-C₈ branched or C₁-C₈ saturated linear alkyl group which is substituted by a -CONH₂ group, which is optionally substituted by a hydroxyl or phenyl group which is optionally substituted by one or more hydroxyls, or a group-COOR₆, where R₆ denotes a C₁-C₆ alkyl group.
R₂ and R₃ = H, and A = group -OR₄, where R₄ denotes H, a C₃-C₆ branched or C₁-C₆ linear saturated alkyl group which is optionally substituted by a -COOH group or a phenyl group.
R₂ and R₃ = H, and A = -NR₈R₉, where R₈ and R₉, which are identical or different, denote H or a C₃-C₆ branched or C₁-C₆ linear saturated alkyl group or a phenyl group;
it being possible for R₈ and R₉ to form, with the nitrogen which carries them, a heterocycle which has 5 or 6 members and may contain an oxygen atom, said heterocycle being optionally substituted by a C₁-C₆ hydrocarbon chain optionally containing one or more radicals selected from hydroxyl or C₁-C₄ alkoxy.
R₂ and R₃ = H, and A = C₃-C₆ branched or C₂-C₆ linear alkyl group interrupted by a -CONH-group and substituted by a COOH group.
R₂ and R₃ = H, and A= C₅-C₆ cyclic alkyl group interrupted by a -CONH- group.
R₂ and R₃ = H, and A = C₅-C₆ cyclic alkyl group interrupted by an oxygen atom.
R₂ and R₃ = H, and A = radical of formula (II) as described above in which X denotes a C₅-C₈ cyclic or C₃-C₆ branched or C₁-C₆ linear saturated hydrocarbon chain or a phenylene group, which is optionally substituted by one or more hydroxyl groups.

Among these compounds, more particular preference is given to the following compounds:

| **No** | **Structure** | **Chemical name** |
|---|---|---|
| 1 | 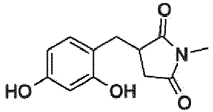 | 3-(2,4-dihydroxybenzy))-1-methylpyrrolidine-2,5-dione |
| 2 | 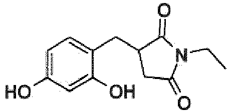 | 3-(2,4-dihydroxybenzyl)-1-ethylpyrrolidine-2,5-dione |
| 3 | 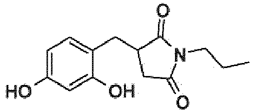 | 3-(2,4-dihydroxybenzyl)-1-propylpyrrolidine-2,5-dione |
| 4 | 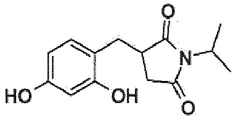 | 3-(2,4-dihydroxybenzyl)-1-isopropylpyrrolidine-2,5-dione |
| 5 | 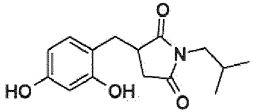 | 3-(2,4-dihydroxybenzyl)-1-isobutylpyrrolidine-2,5-dione |
| 6 | 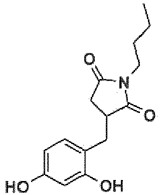 | 3-(2,4-dihydroxybenzyl)-1-butylpyrrolidine-2,5-Dione |
| 7 | 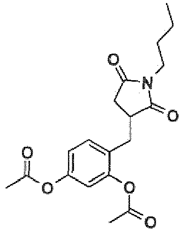 | 4-[(1-butyl-2,5-dioxopyrrolidin-3-yl)methyl]benzene-1,3-diyl diacetate |
| 8 | 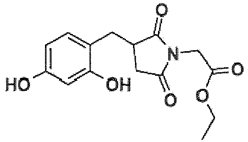 | ethyl [3-(2,4-dihydroxybenzyl)-2,5-dioxopyrrolidin-1-yl]acetate |
| 9 | 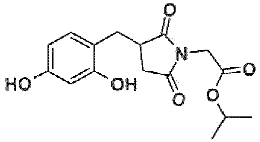 | isopropyl [3-(2,4-dihydroxybenzyl) -2,5-dioxopyrrolidin-1-yl]acetate |
| 10 | 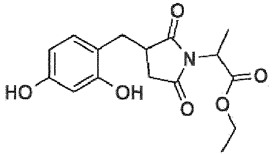 | ethyl 2-[3-(2,4-dihydroxybenzyl)-2,5-dioxopyrrolidin-1-yl]propanoate |
| 11 | 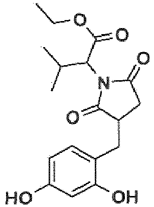 | ethyl 2-[3-(2,4-dihydroxybenzyl)-2,5-dioxopyrrolidin-1-yl]-3-methylbutanoate |

and also their salts.

The above compounds can be prepared in accordance with, for example, the process described in WO 2012/079938, the entirety of which is incorporated herein by reference.

The resorcinol derivatives used in the present invention preferably include 4-alkylresorcinols, in particular 4-n-butylresorcinol, phenylethyl resorcinol, and 4-(tetrahydro-2H-pyran-4-yl) benzene-1,3-diol.

The cinnamaldehyde derivatives used in the present invention are selected from trans-ferulic acid, p-coumaric acid, and coniferylaldehyde.

Preferably, the active ingredients are phenylethyl resorcinol, 4-(tetrahydro-2H-pyran-4-yl) benzene-1,3-diol, or a combination thereof.

In one embodiment of the present invention, the active ingredient has a solubility of at least 0.01g, preferably 0.05g, more preferably 0.1g relative to 100g of the oil at room temperature (25°C) and under atmospheric pressure (760 mmHg). The maximum oil solubility of the active ingredient is not limited, but for example, the active ingredient has a solubility of less than 50g, preferably less than 40g, and more preferably less than 30g relative to 100g of the oil at room temperature and under atmospheric pressure.

Another embodiment of the present invention, the active ingredient has a solubility of less than 1g, preferably less than 0.5g, more preferably less than 0.1g, in particular less than 0.01g relative to 100 g of water at room temperature and under atmospheric pressure.

For the present invention, a saturation degree of the active ingredient in the oil phase is preferably more than 0.90, more preferably more than 0.92, and even more preferably more than 0.95, and in particular more than 0.97. In the present invention, the saturation degree is defined as the ratio between the concentration of the active ingredient in the oil phase and the maximum solubility of the active ingredients in the oil phase measured at room temperature and under atmospheric pressure.

The maximum solubility of the active ingredients in the oil phase can be measured in any way which is known in the art. For example, an excess amount of the active ingredient is dissolved in the oil and mixed vigorously at elevated temperature, and then this sample is cooled down to room temperature and maintained for several hours to days. This mixture is centrifuged to separate an oil phase and the concentration of the active ingredient in the separated oil phase is measured using UV spectroscopy.

The concentration of the active ingredients in the oil phase in the composition according to the present invention can be measured in any way which is known in the art. For example, after preparing the composition according to the present invention, the oil phase is separated by centrifuging at 14,000 rpm for 20 min, and then the separated oil phase is collected and the concentration of the active ingredients in the oil phase is measured by UV spectroscopy.

In the representative examples of the active ingredients of the present invention, the maximum solubilities of phenylethyl resorcinol and 4-(tetrahydro-2H-pyran-4-yl) benzene-1,3-diol in various oils measured at room temperature and under atmospheric pressure are shown below.

| Name of oil | Phenylethyl Resorcinol | 4-(tetrahydro-2H-pyran-4-yl)benzene-1,3-diol |
|---|---|---|
| Isopropyl Lauroyl Sarcosinate | 42% | 9% |
| Isopropyl Myristate | 36% | < 1 % |
| Ethyl Hexyl Palmitate | 22 | <1% |
| Castor oil | N.A | 1.5 |
| Capryl/Caprylic Triglyceride | N.A | <1% |
| Isododecane | 0.043% | N.A |
| Sesamum Indicum (Sesame) Seed Oil | 23% | N.A |

| | | |
|---|---|---|
| N.A. indicates "not analyzed" | | |

The amount of the (b) active ingredient in the composition according to the present invention is not limited, and may range from 0.01 to 5% by weight, preferably from 0.05 to 3% by weight, more preferably from 0.1 to 2% by weight, relative to the total weight of the composition. The amount of the (b) active ingredient in the oil phase ranges from 0.1 to 20% by weight, preferably from 1 to 15% by weight, more preferably from 3 to 10% by weight, relative to the total weight of the oil phase.

### Aqueous Phase

The aqueous phase of the O/W emulsion composition according to the present invention includes (c) water. The amount of the water in the composition is from 60 to 99 % by weight relative to the total weight of the composition. It can be more than 60% by weight, preferably more than 70% by weight, and in particular more than 80% by weight. The composition can include less than 99% by weight of water, preferably less than 98% by weight of water, and more preferably less than 97% by weight of water.

The aqueous phase of the present invention substantially does not include water soluble alcohols. The term "water soluble alcohol" used herein means alcohol which can dissolve in an amount of 1 g or more, 5g or more, or 10g or more in 100 mL water at room temperature and under atmospheric pressure. The aqueous phase comprises less than 5% by weight, preferably less than 1% by weight, and more preferably less than 0.1% by weight of water soluble alcohol, relative to the total amount of the aqueous phase, and in particular, the aqueous phase comprises no water soluble alcohol.

The water soluble alcohols include, but are not limited to, linear or branched lower mono-alcohols having from 1 to 8 carbon atoms, such as ethanol, propanol, butanol, isopropanol, and isobutanol, polyols such as ethylene glycol, diethylene glycol, triethylene glycol, propylene glycol, dipropylene glycol, glycol, glycerin, triethylene glycol, erythritol, and sugar alcohols such as sorbitol.

One assumable hypothesis for the present invention being capable of improving a penetration property of the active ingredient through a keratinous substance is that an elimination of alcohols from the aqueous phase can prevent the active ingredients dissolved in the oil phase from transferring to the aqueous phase, and thus the oil phase can maintain a high concentration of the active ingredients.

### (Surfactant)

The composition according to the present invention comprises at least one (d) surfactant selected from nonionic surfactants. Two or more (d) surfactants may be used in combination.

Thus, a single type of a surfactant or a combination of different types of surfactants may be used. Also, the composition can further comprise anionic surfactants.

Preferably, the (d) surfactants used in the composition have an HLB (Hydrophilic Lipophilic Balance) value of from 8 to 22, preferably from 9 to 18, and more preferably from 10 to 14. If two or more surfactants are used, the HLB value can be determined by the weight average of the HLB values of all the surfactants.

### Nonionic Surfactant

The nonionic surfactants are compounds well known in themselves (see, e.g., in this regard, "Handbook of Surfactants" by M. R. Porter, Blackie & Son publishers (Glasgow and London), 1991, pp. 116-178). Thus, they can, for example, be chosen from alcohols, alpha-diols, alkylphenols and esters of fatty acids, these compounds being ethoxylated, propoxylated or glycerolated and having at least one fatty chain comprising, for example, from 8 to 30 carbon atoms, it being possible for the number of ethylene oxide or propylene oxide groups to range from 2 to 50, and for the number of glycerol groups to range from 1 to 30. Maltose derivatives may also be mentioned. Non-limiting mention may also be made of copolymers of ethylene oxide and/or of propylene oxide; condensates of ethylene oxide and/or of propylene oxide with fatty alcohols; polyethoxylated fatty amides comprising, for example, from 2 to 30 mol of ethylene oxide; polyglycerolated fatty amides comprising, for example, from 1.5 to 5 glycerol groups, such as from 1.5 to 4; ethoxylated fatty acid esters of sorbitan comprising from 2 to 30 mol of ethylene oxide; ethoxylated oils of plant origin; fatty acid esters of sucrose; fatty acid esters of polyethylene glycol; polyethoxylated fatty acid mono or diesters of glycerol (C₆-C₂₄)alkylpolyglycosides; N-(C₆-C₂₄)alkylglucamine derivatives; amine oxides such as (C₁₀-C₁₄)alkylamine oxides or N-(C₁₀-C₁₄)acylaminopropylmorpholine oxides; silicone surfactants; and mixtures thereof.

The nonionic surfactants may preferably be chosen from monooxyalkylenated, polyoxyalkylenated, monoglycerolated or polyglycerolated nonionic surfactants. The oxyalkylene units are more particularly oxyethylene or oxypropylene units, or a combination thereof, and are preferably oxyethylene units.

Examples of monooxyalkylenated or polyoxyalkylenated nonionic surfactants that may be mentioned include:
monooxyalkylenated or polyoxyalkylenated (C₈-C₂₄)alkylphenols,
saturated or unsaturated, linear or branched, monooxyalkylenated or polyoxyalkylenated C₈-C₃₀ alcohols,
saturated or unsaturated, linear or branched, monooxyalkylenated or polyoxyalkylenated C₈-C₃₀ amides,
esters of saturated or unsaturated, linear or branched, C₈-C₃₀ acids and of polyalkylene glycols,
monooxyalkylenated or polyoxyalkylenated esters of saturated or unsaturated, linear or branched, C₈-C₃₀ acids and of sorbitol,
saturated or unsaturated, monooxyalkylenated or polyoxyalkylenated plant oils,
condensates of ethylene oxide and/or of propylene oxide, inter alia, alone or as mixtures.

The surfactants preferably contain a number of moles of ethylene oxide and/or of propylene oxide of between 1 and 100 and most preferably between 2 and 50. Advantageously, the nonionic surfactants do not comprise any oxypropylene units.

According to one of the embodiments of the present invention, the polyoxyalkylenated nonionic surfactants are chosen from polyoxyethylenated fatty alcohol (polyethylene glycol ether of fatty alcohol) and polyoxyethylenated fatty ester (polyethylene glycol ester of fatty acid).

Examples of polyoxyethylenated fatty alcohol (or C₈-C₃₀ alcohols) that may be mentioned include the adducts of ethylene oxide with lauryl alcohol, especially those containing from 7 to 50 oxyethylene units and more particularly those containing from 6 to 12 oxyethylene units (Laureth-6 to Laureth-12, as the CTFA names); the adducts of ethylene oxide with behenyl alcohol, especially those containing from 5 to 50 oxyethylene units (Beheneth-5 to Beheneth-50, as the CTFA names); the adducts of ethylene oxide with cetearyl alcohol (mixture of cetyl alcohol and stearyl alcohol), especially those containing from 7 to 30 oxyethylene units (Ceteareth-7 to Ceteareth-30, as the CTFA names); the adducts of ethylene oxide with cetyl alcohol, especially those containing from 7 to 30 oxyethylene units (Ceteth-7 to Ceteth-30, as the CTFA names); the adducts of ethylene oxide with stearyl alcohol, especially those containing from 7 to 30 oxyethylene units (Steareth-7 to Steareth-30, as the CTFA names); the adducts of ethylene oxide with isostearyl alcohol, especially those containing from 8 to 50 oxyethylene units (Isosteareth-8 to Isosteareth-50, as the CTFA names); and mixtures thereof.

As examples of monoglycerolated or polyglycerolated nonionic surfactants, monoglycerolated or polyglycerolated C₈-C₄₀ alcohols are preferably used.

In particular, the monoglycerolated or polyglycerolated C₈-C₄₀ alcohols correspond to the following formula:

RO-[CH₂-CH(CH₂OH)-O]ₘ-H or RO-[CH(CH₂OH)-CH₂O]ₘ-H

in which R represents a linear or branched C₈-C₄₀ and preferably C₈-C₃₀ alkyl or alkenyl radical, and m represents a number ranging from 1 to 30 and preferably from 1.5 to 10.

As examples of compounds that are suitable in the context of the present invention, mention may be made of lauryl alcohol containing 4 mol of glycerol (INCI name: Polyglyceryl-4 Lauryl Ether), lauryl alcohol containing 1.5 mol of glycerol, oleyl alcohol containing 4 mol of glycerol (INCI name: Polyglyceryl-4 Oleyl Ether), oleyl alcohol containing 2 mol of glycerol (INCI name: Polyglyceryl-2 Oleyl Ether), cetearyl alcohol containing 2 mol of glycerol, cetearyl alcohol containing 6 mol of glycerol, oleocetyl alcohol containing 6 mol of glycerol, and octadecanol containing 6 mol of glycerol.

The alcohol may represent a mixture of alcohols in the same way that the value of m represents a statistical value, which means that, in a commercial product, several species of polyglycerolated fatty alcohol may coexist in the form of a mixture.

Among the monoglycerolated or polyglycerolated alcohols, it is preferable to use the C₈/C₁₀ alcohol containing 1 mol of glycerol, the C₁₀/C₁₂ alcohol containing 1 mol of glycerol and the C₁₂ alcohol containing 1.5 mol of glycerol.

The monoglycerolated or polyglycerolated C₈-C₄₀ fatty esters may correspond to the following formula:

R'O-[CH₂-CH(CH₂OR‴)-O]ₘ-R" or R'O-[CH(CH₂OR‴)-CH₂O]ₘ-R"

in which each of R', R" and R‴ independently represents a hydrogen atom, or a linear or branched C₈-C₄₀ and preferably C₈-C₃₀ alkyl-CO- or alkenyl-CO-radical, with the proviso that at least one of R', R" and R‴ is not a hydrogen atom, and m represents a number ranging from 1 to 30 and preferably from 1.5 to 10.

Examples of polyoxyethylenated fatty esters that may be mentioned include the adducts of ethylene oxide with esters of lauric acid, palmitic acid, stearic acid or behenic acid, and mixtures thereof, especially those containing from 9 to 100 oxyethylene units, such as PEG-9 to PEG-50 laurate (as the CTFA names: PEG-9 laurate to PEG-50 laurate); PEG-9 to PEG-50 palmitate (as the CTFA names: PEG-9 palmitate to PEG-50 palmitate); PEG-9 to PEG-50 stearate (as the CTFA names: PEG-9 stearate to PEG-50 stearate); PEG-9 to PEG-50 palmitostearate; PEG-9 to PEG-50 behenate (as the CTFA names: PEG-9 behenate to PEG-50 behenate); polyethylene glycol 100 EO monostearate (CTFA name: PEG-100 stearate); and mixtures thereof.

According to one of the embodiments according to the present invention, the nonionic surfactant may be selected from esters of polyols with fatty acids with a saturated or unsaturated chain containing for example from 8 to 24 carbon atoms, preferably 12 to 22 carbon atoms, and polyoxyalkylenated derivatives thereof, preferably containing from 10 to 200, and more preferably from 10 to 100 oxyalkylene units, such as mono glyceryl esters or poly glyceryl esters of a C₈-C₂₄, preferably C₁₂-C₂₂, fatty acid or acids and polyoxyalkylenated derivatives thereof, preferably containing from 10 to 200, and more preferably from 10 to 100 oxyalkylene units; sorbitol esters of a C₈-C₂₄, preferably C₁₂-C₂₂, fatty acid or acids and polyoxyalkylenated derivatives thereof, preferably containing from 10 to 200, and more preferably from 10 to 100 oxyalkylene units; sugar (sucrose, maltose, glucose, fructose, and/or alkylglycose) esters of a C₈-C₂₄, preferably C₁₂-C₂₂, fatty acid or acids and polyoxyalkylenated derivatives thereof, preferably containing from 10 to 200, and more preferably from 10 to 100 oxyalkylene units; ethers of fatty alcohols; ethers of sugar and a C₈-C₂₄, preferably C₁₂-C₂₂, fatty alcohol or alcohols; and mixtures thereof.

As mono glyceryl esters of fatty acids, glyceryl stearate (glyceryl mono-, di- and/or tristearate) (CTFA name: glyceryl stearate) or glyceryl ricinoleate and mixtures thereof can be cited, and as polyoxyalkylenated derivatives thereof, mono-, di- or triester of fatty acids with a polyoxyalkylenated glycerol (mono-, di- or triester of fatty acids with a polyalkylene glycol ether of glycerol), preferably polyoxyethylenated glyceryl stearate (mono-, di- and/or tristearate), such as PEG-20 glyceryl stearate (mono-, di- ,tristearate and/or triisostearate) can be cited. Preferably, the polyoxyalkylenated derivative of mono glyceryl ester of fatty acids includes 10 to 40 oxyethylene units, such as PEG-20 glyceryl triisostearate.

Mixtures of these surfactants, such as for example the product containing glyceryl stearate and PEG-100 stearate, marketed under the name ARLACEL 165 by Uniqema, and the product containing glyceryl stearate (glyceryl mono- and distearate) and potassium stearate marketed under the name TEGIN by Goldschmidt (CTFA name: glyceryl stearate SE), can also be used.

As polyglyceryl esters of (a) fatty acid(s), mention be made of the product containing 2 to 10 glycerol units, such as polyglyceryl monolaurate, oleate, myristate, caprylate, or stearate comprising 2 to 10 glycerol units, polyglyceryl mono(iso)stearate comprising 2 to 10 glycerol units, polyglyceryl dioleate comprising 2 to 10 glycerol units, polyglyceryl dilaurate comprising 2 to 10 glycerol units, polyglyceryl dimyristate comprising 2 to 10 glycerol units, polyglyceryl trimyristate comprising 2 to 10 glycerol units, polyglyceryl trioleate comprising 2 to 10 glycerol units, and polyglyceryl tricaprylate comprising 2 to 10 glycerol units.

The sorbitol esters of C₈-C₂₄ fatty acids and polyoxyalkylenated derivatives thereof can be selected from sorbitan palmitate, sorbitan isostearate, sorbitan trioleate and esters of fatty acids and alkoxylated sorbitan containing for example from 20 to 100 EO, such as for example sorbitan monostearate (CTFA name: sorbitan stearate), sold by the company ICI under the name Span 60, sorbitan monopalmitate (CTFA name: sorbitan palmitate), sold by the company ICI under the name Span 40, and sorbitan tristearate 20 EO (CTFA name: polysorbate 65), sold by the company ICI under the name Tween 65, polyethylene sorbitan trioleate (polysorbate 85) or the compounds marketed under the trade names Tween 20 or Tween 60 by Uniqema.

As esters of fatty acids and glucose or alkylglucose, glucose palmitate, alkylglucose sesquistearates such as methylglucose sesquistearate, alkylglucose palmitates such as methylglucose or ethylglucose palmitate, methylglucoside fatty esters, the diester of methylglucoside and oleic acid (CTFA name: Methyl glucose dioleate), the mixed ester of methylglucoside and the mixture of oleic acid/hydroxystearic acid (CTFA name: Methyl glucose dioleate/hydroxystearate), the ester of methylglucoside and isostearic acid (CTFA name: Methyl glucose isostearate), the ester of methylglucoside and lauric acid (CTFA name: Methyl glucose laurate), the mixture of monoester and diester of methylglucoside and isostearic acid (CTFA name: Methyl glucose sesqui-isostearate), the mixture of monoester and diester of methylglucoside and stearic acid (CTFA name: Methyl glucose sesquistearate) and in particular the product marketed under the name Glucate SS by AMERCHOL, and mixtures thereof can be cited.

As ethoxylated ethers of fatty acids and glucose or alkylglucose, ethoxylated ethers of fatty acids and methylglucose, and in particular the polyethylene glycol ether of the diester of methylglucose and stearic acid with about 20 moles of ethylene oxide (CTFA name: PEG-20 methyl glucose distearate) such as the product marketed under the name Glucam E-20 distearate by AMERCHOL, the polyethylene glycol ether of the mixture of monoester and diester of methyl-glucose and stearic acid with about 20 moles of ethylene oxide (CTFA name: PEG-20 methyl glucose sesquistearate) and in particular the product marketed under the name Glucamate SSE-20 by AMERCHOL and that marketed under the name Grillocose PSE-20 by GOLDSCHMIDT, and mixtures thereof, can for example be cited.

As sucrose esters, saccharose palmito-stearate, saccharose stearate and saccharose monolaurate can for example be cited.

As sugar ethers, alkylpolyglucosides can be used, and for example, ethers of a sugar and of C₈-C₂₄ fatty alcohols including decylglucoside such as the product marketed under the name MYDOL 10 by Kao Chemicals, the product marketed under the name PLANTAREN 2000 by Henkel, and the product marketed under the name ORAMIX NS 10 by Seppic, caprylyl/capryl glucoside such as the product marketed under the name ORAMIX CG 110 by Seppic or under the name LUTENSOL GD 70 by BASF, laurylglucoside such as the products marketed under the names PLANTAREN 1200 N and PLANTACARE 1200 by Henkel, coco-glucoside such as the product marketed under the name PLANTACARE 818/UP by Henkel, cetostearyl glucoside possibly mixed with cetostearyl alcohol, marketed for example under the name MONTANOV 68 by Seppic, under the name TEGO-CARE CG90 by Goldschmidt and under the name EMULGADE KE3302 by Henkel, arachidyl glucoside, for example in the form of the mixture of arachidyl and behenyl alcohols and arachidyl glucoside marketed under the name MONTANOV 202 by Seppic, cocoylethylglucoside, for example in the form of the mixture (35/65) with cetyl and stearyl alcohols, marketed under the name MONTANOV 82 by Seppic, and mixtures thereof can in particular be cited.

Mixtures of glycerides of alkoxylated plant oils such as mixtures of ethoxylated (200 EO) palm and copra (7 EO) glycerides can also be cited.

The nonionic surfactant according to the present invention preferably contains alkenyl or a branched C₁₂-C₂₂ acyl chain such as an oleyl or isostearyl group.

According to one of the embodiments according to the present invention, the nonionic surfactant may be selected from copolymers of ethylene oxide and of propylene oxide, in particular copolymers of the following formula:

HO(C₂H₄O)ₐ(C₃H₆O)_{b}(C₂H₄O)_{c}H

in which a, b and c are integers such that a+c ranges from 2 to 100 and b ranges from 14 to 60, and mixtures thereof.

According to one of the embodiments according to the present invention, the nonionic surfactant may be selected from silicone surfactants. Non-limiting mention may be made of those disclosed in documents US-A-5364633 and US-A-5411744.

The silicone surfactant may preferably be a compound of formula (I): 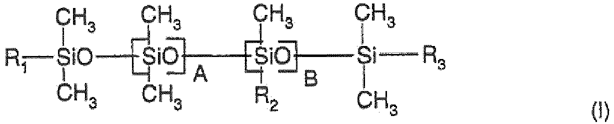 in which:
R₁, R₂ and R₃, independently of each other, represent a C₁-C₆ alkyl radical or a radical -(CH₂)ₓ-(OCH₂CH₂)_{y}-(OCH₂CH₂CH₂)_{z}-OR₄, at least one radical R₁, R₂ or R₃ not being an alkyl radical; R₄ being a hydrogen, an alkyl radical or an acyl radical;
A is an integer ranging from 0 to 200;
B is an integer ranging from 0 to 50; with the proviso that A and B are not simultaneously equal to zero;
x is an integer ranging from 1 to 6;
y is an integer ranging from 1 to 30;
z is an integer ranging from 0 to 5.

According to one preferred embodiment of the present invention, in the compound of formula (I), the alkyl radical is a methyl radical, x is an integer ranging from 2 to 6 and y is an integer ranging from 4 to 30.

As examples of silicone surfactants of formula (I), mention may be made of the compounds of formula (II): 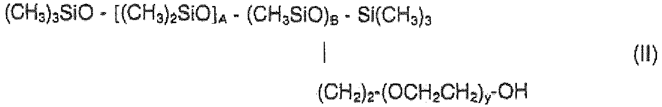 in which A is an integer ranging from 20 to 105, B is an integer ranging from 2 to 10 and y is an integer ranging from 10 to 20.

As examples of silicone surfactants of formula (I), mention may also be made of the compounds of formula (III):

H-(OCH₂CH₂)_{y}-(CH₂)₃-[(CH₃)₂SiO]_{A'}-(CH₂)₃-(OCH₂CH₂)_{y}-OH (III)

in which A' and y are integers ranging from 10 to 20.

Compounds of the present invention which may be used are those sold by the company Dow Corning under the names DC 5329, DC 7439-146, DC 2-5695 and Q4-3667. The compounds DC 5329, DC 7439-146 and DC 2-5695 are compounds of formula (III) in which, respectively, A is 22, B is 2 and y is 12; A is 103, B is 10 and y is 12; A is 27, B is 3 and y is 12. The compound Q4-3667 is a compound of formula (III) in which A is 15 and y is 13.

Preferably, the nonionic surfactant used in the composition according to the present invention can be selected from a group consisting of, ethers of a sugar and of C₈-C₂₄ fatty alcohols, such as caprylyl/capryl glucoside, polyoxyethylenated fatty alcohol containing from 6 to 12 oxyethylene units, such as Laureth-9, polyoxyalkylenated derivative of mono glyceryl ester of a fatty acid, such as PEG-20 glyceryl triisostearate, and polyglyceryl esters of a fatty acid, such as polyglyceryl-2 oleate.

### Anionic Surfactant

The anionic surfactants may be chosen in particular from phosphates and alkyl phosphates, carboxylates, sulphosuccinates, amino acid derivatives, alkyl sulphates, alkyl ether sulphates, sulphonates, isethionates, taurates, polyoxyethylene alkyl ether carboxylic acids, alkyl sulphoacetates, polypeptides, and their mixtures.
1) Mention may be made, as phosphates and alkyl phosphates, for example, of monoalkyl phosphates and dialkyl phosphates, such as lauryl monophosphate, sold under the name MAP 20^{®} by Kao Chemicals, the potassium salt of dodecyl phosphate, the mixture of mono- and diesters (predominantly diester) sold under the name Crafol AP-31^{®} by Cognis, the mixture of octyl phosphate monoester and diester, sold under the name Crafol AP-20^{®} by Cognis, the mixture of ethoxylated (7 mol of EO) 2-butyloctyl phosphate monoester and diester, sold under the name Isofol 12 7 EO-Phosphate Ester^{®} by Condea, the potassium or triethanolamine salt of mono(C₁₂-C₁₃)alkyl phosphate, sold under the references Arlatone MAP 230K-40^{®} and Arlatone MAP 230T-60^{®} by Uniqema, potassium lauryl phosphate, sold under the name Dermalcare MAP XC-99/09^{®} by Rhodia Chimie, and potassium cetyl phosphate, sold under the name Arlatone MAP 160K by Uniqema.
2) Mention may be made, as carboxylates, of:
   - amido ether carboxylates (AEC), such as sodium lauryl amido ether carboxylate (3 EO), sold under the name Akypo Foam 30^{®} by Kao Chemicals;
   - polyoxyethylenated carboxylic acid salts, such as oxyethylenated (6 EO) sodium lauryl ether carboxylate (65/25/10 C₁₂-C₁₄-C₁₆), sold under the name Akypo Soft 45 NV^{®} by Kao Chemicals, polyoxyethylenated and carboxymethylated fatty acids originating from olive oil, sold under the name Olivem 400^{®} by Biologia E Tecnologia, or oxyethylenated (6 EO) sodium tridecyl ether carboxylate, sold under the name Nikkol ECTD-6NEX^{®} by Nikkol; and
   - salts of fatty acids (soaps) having a C₆ to C₂₂ alkyl chain which are neutralized with an organic or inorganic base, such as potassium hydroxide, sodium hydroxide, triethanolamine, N-methylglucamine, lysine and arginine.
3) Mention may in particular be made, as amino acid derivatives, of alkali salts of amino acids, such as:
   - sarcosinates, such as sodium lauroyl sarcosinate, sold under the name Sarkosyl NL 97^{®} by Ciba or sold under the name Oramix L 30^{®} by Seppic, sodium myristoyl sarcosinate, sold under the name Nikkol Sarcosinate MN^{®} by Nikkol, or sodium palmitoyl sarcosinate, sold under the name Nikkol Sarcosinate PN^{®} by Nikkol;
   - alaninates, such as sodium N-lauroyl-N-methylamidopropionate, sold under the name Sodium Nikkol Alaninate LN 30^{®} by Nikkol or sold under the name Alanone ALE^{®} by Kawaken, or triethanolamine N-lauroyl-N-methylalanine, sold under the name Alanone ALTA^{®} by Kawaken;
   - glutamates, such as triethanolamine monococoyl glutamate, sold under the name Acylglutamate CT-12^{®} by Ajinomoto, triethanolamine lauroyl glutamate, sold under the name Acylglutamate LT-12^{®} by Ajinomoto;
   - aspartates, such as the mixture of triethanolamine N-lauroyl aspartate and triethanolamine N-myristoyl aspartate, sold under the name Asparack^{®} by Mitsubishi;
   - glycine derivatives (glycinates), such as sodium N-cocoyl glycinate, sold under the names Amilite GCS-12^{®} and Amilite GCK 12 by Ajinomoto;
   - citrates, such as the citric monoester of oxyethylenated (9 mol) coco alcohols, sold under the name Witconol EC 1129 by Goldschmidt; and
   - galacturonates, such as sodium dodecyl D-galactoside uronate, sold by Soliance.
4) Mention may be made, as sulphosuccinates, for example, of oxyethylenated (3 EO) lauryl (70/30 C₁₂/C₁₄) alcohol monosulphosuccinate, sold under the names Setacin 103 Special^{®} and Rewopol SB-FA 30 K 4^{®} by Witco, the disodium salt of a hemisulphosuccinate of C₁₂-C₁₄ alcohols, sold under the name Setacin F Special Paste^{®} by Zschimmer Schwarz, oxyethylenated (2 EO) disodium oleamidosulphosuccinate, sold under the name Standapol SH 135^{®} by Cognis, oxyethylenated (5 EO) lauramide monosulphosuccinate, sold under the name Lebon A-5000^{®} by Sanyo, the disodium salt of oxyethylenated (10 EO) lauryl citrate monosulphosuccinate, sold under the name Rewopol SB CS 50^{®} by Witco, or ricinoleic monoethanolamide monosulphosuccinate, sold under the name Rewoderm S 1333^{®} by Witco. Use may also be made of polydimethylsiloxane sulphosuccinates, such as disodium PEG-12 dimethicone sulphosuccinate, sold under the name Mackanate-DC 30 by MacIntyre.
5) Mention may be made, as alkyl sulphates, for example, of triethanolamine lauryl sulphate (CTFA name: TEA lauryl sulphate), such as the product sold by Huntsman under the name Empicol TL40 FL or the product sold by Cognis under the name Texapon T42, which products are at 40% in aqueous solution. Mention may also be made of ammonium lauryl sulphate (CTFA name: ammonium lauryl sulphate), such as the product sold by Huntsman under the name Empicol AL 30FL, which is at 30% in aqueous solution.
6) Mention may be made, as alkyl ether sulphates, for example, of sodium lauryl ether sulphate (CTFA name: sodium laureth sulphate), such as that sold under the names Texapon N40 and Texapon AOS 225 UP by Cognis, or ammonium lauryl ether sulphate (CTFA name: ammonium laureth sulphate), such as that sold under the name Standapol EA-2 by Cognis.
7) Mention may be made, as sulphonates, for example, of α-olefinsulphonates, such as sodium α-olefinsulphonate (C₁₄-C₁₆), sold under the name Bio-Terge AS-40^{®} by Stepan, sold under the names Witconate AOS Protégé^{®} and Sulframine AOS PH 12^{®} by Witco or sold under the name Bio-Terge AS-40 CG^{®} by Stepan, secondary sodium olefinsulphonate, sold under the name Hostapur SAS 30^{®} by Clariant; or linear alkylarylsulphonates, such as sodium xylenesulphonate, sold under the names Manrosol SXS30^{®}, Manrosol SXS40^{®} and Manrosol SXS93^{®} by Manro.
8) Mention may be made, as isethionates, of acylisethionates, such as sodium cocoylisethionate, such as the product sold under the name Jordapon CI P^{®} by Jordan.
9) Mention may be made, as taurates, of the sodium salt of palm kernel oil methyltaurate, sold under the name Hostapon CT Paté^{®} by Clariant; N-acyl-N-methyltaurates, such as sodium N-cocoyl-N-methyltaurate, sold under the name Hostapon LT-SF^{®} by Clariant or sold under the name Nikkol CMT-30-T^{®} by Nikkol, sodium palmitoyl methyltaurate, sold under the name Nikkol PMT^{®} by Nikkol, or sodium steraroyl methyltaurate, sold under the name Sunsoft O-30S by Taiyo Kagaku.
10) Mention may be made of polyoxyethylene alkyl ether carboxylic acids, such as compounds corresponding to formula (IV):

   RO[CH₂O]ᵤ[(CH₂)ₓCH(R')(CH₂)_{y}(CH₂)_{z}O]ᵥ[CH₂CH₂O]_{w}CH₂COOH (IV)

   wherein:
   R is a hydrocarbon radical containing from 6 to 40 carbon atoms;
   u, v and w, independently of one another, represent numbers of from 0 to 60;
   x, y and z, independently of one another, represent numbers of from 0 to 13;
   R' represents hydrogen, alkyl, preferably C₁-C₁₂ alkyl; and
   the sum of x+y+z is 0 or more.

In formula (IV), R is linear or branched, acyclic or cyclic, saturated or unsaturated, aliphatic or aromatic, substituted or unsubstituted. As examples of the substituent, mention may be made of a monovalent functional group such as a halogen atom, a hydroxyl group, a C₁-C₆ alkoxy group, an amino group, a C₁-C₆ alkylamino group, a C₁-C₆ dialkylamino group, a nitro group, a carbonyl group, an acyl group, a carboxyl group, a cyano group and the like. Typically, R is a linear or branched, acyclic C₆-C₄₀ alkyl or alkenyl group or a C₁-C₄₀ alkyl phenyl group, more typically a C₈-C₂₄ alkyl or alkenyl group or a C₄-C₂₀ alkyl phenyl group, and even more typically a C₁₀-C₁₈ alkyl group or alkenyl group or a C₆-C₁₆ alkyl phenyl group, which may be substituted; u, v, w, independently of one another, is typically a number from 2 to 20, more typically a number from 3 to 17, and most typically a number from 5 to 15; x, y, z, independently of one another, is typically a number from 2 to 13, more typically a number from 1 to 10, and most typically a number from 0 to 8;

The polyoxyethylene alkyl ether carboxylic acids, corresponding to formula (IV) can be obtained by alkoxylation of alcohols ROH with ethylene oxide as the sole alkoxide or with several alkoxides and subsequent oxidation. The numbers u, v, and w each represent the degree of alkoxylation. Whereas, on a molecular level, the numbers u, v and w and the total degree of alkoxylation can only be integers, including zero, on a macroscopic level they are mean values in the form of broken numbers. The fatty ether carboxylic acids may include polyoxyalkylenated (C₆-C₃₀)alkyl ether carboxylic acid and their salts, more specifically polyoxyethylenated (C₆-C₃₀) alkyl ether carboxylic acids and their salts; polyoxyalkylenated (C₆-C₃₀)alkylaryl ether carboxylic acids and their salts; and polyoxyalkylenated (C₆-C₃₀)alkylamido ether carboxylic acids. Preferably, the fatty ether carboxylic acids are polyoxyethylene (3) to (17) lauryl ether carboxylic acid.

Suitable polyoxyethylene alkyl ether carboxylic acids, include, but are not limited to, the following representatives referred to by their INCI names: Butoxynol-5 to 19 Carboxylic Acid, Capryleth-4 to 25 Carboxylic Acid, Coceth-7 Carboxylic Acid, C₉₋₁₅ Pareth-6 to 8 Carboxylic Acid, Deceth-7 Carboxylic Acid, Laureth-3 to 17 Carboxylic Acid, in particular Laureth-5 Carboxylic Acid, such as the product marketed under the name "Akypo RLM 45 CA" by Kao Chemical Co., PPG-6-Laureth-6 Carboxylic Acid, PPG-8-Steareth-7 Carboxylic Acid, Myreth-3 to 5 Carboxylic Acid, Nonoxynol-5 to 10 Carboxylic Acid, Octeth-3 Carboxylic Acid, Octoxynol-20 Carboxylic Acid, Oleth-3 t o10 Carboxylic Acid, PPG-3-Deceth-2 Carboxylic Acid, Capryleth-2 Carboxylic Acid, Ceteth-13 Carboxylic Acid, Deceth-2 Carboxylic Acid, Hexeth-4 Carboxylic Acid, Isosteareth-6 to 11 Carboxylic Acid, Trudeceth-3 to 12 Carboxylic Acid, Trideceth-3 to 19 Carboxylic Acid, Undeceth-5 Carboxylic Acid, and mixtures thereof.

Preferably, the anionic surfactants used in the composition according to the present invention are sarcosinates, such as sodium lauroyl sarcosinate and polyoxyethylene alkyl ether carboxylic acids, such as polyoxyethylene (3) to (17) lauryl ether carboxylic acid, in particular Laureth-5 Carboxylic Acid.

The amount of the (d) surfactants in the composition according to the present invention is not limited, and may range from 0.1 to 20% by weight, preferably from 0.5 to 15% by weight, more preferably from 1 to 10% by weight, relative to the total weight of the composition.

### (Other Ingredients)

The composition according to the present invention may also comprise at least one additional ingredient.

The composition according to the present invention may comprise at least one gelling agent. The gelling agent usable in the composition according to the present invention may include water soluble polymers such as, for example, high molecular weight crosslinked homopolymers of acrylic acid, and Acrylates/C₁₀₋₃₀ Alkyl Acrylate Crosspolymer, such as Carbopol^{®} and Pemulen^{®}; anionic acrylate polymers such as Salcare^{®} AST and cationic acrylate polymers such as Salcare^{®} SC96; acrylamidopropylttrimonium chloride/acrylamide; hydroxyethyl methacrylate polymers, Steareth-10 Allyl Ether/Acrylate Copolymer; Acrylates/Beheneth-25 Metacrylate Copolymer, known as Aculyn^{®} 28; glyceryl polymethacrylate, Acrylates/Steareth-20 Methacrylate Copolymer; bentonite; gums such as alginates, carageenans, gum acacia, gum arabic, gum ghatti, gum karaya, gum tragacanth, guar gum; guar hydroxypropyltrimonium chloride, xanthan gum or gellan gum; cellulose derivatives such as sodium carboxymethyl cellulose, hydroxyethyl cellulose, hydroxymethyl carboxyethyl cellulose, hydroxymethyl carboxypropyl cellulose, ethyl cellulose, sulfated cellulose, hydroxypropyl cellulose, methyl cellulose, hydroxypropylmethyl cellulose, microcrystalline cellulose; agar; pectin; gelatin; starch and its derivatives; chitosan and its derivatives such as hydroxyethyl chitosan; polyvinyl alcohol, PVM/MA copolymer, PVM/MA decadiene crosspolymer, poly(ethylene oxide) based gelling agents, sodium carbomer, and mixtures thereof.

The amount of the gelling agent(s) in the composition may be from 0.1 to 20% by weight, preferably from 0.2 to 15% by weight, and more preferably from 1 to 10% by weight, relative to the total weight of the composition.

The composition according to present invention may comprise other additives usually used in cosmetics. The additives may be selected from the group consisting of anionic, cationic, nonionic or amphoteric polymers; cationic or amphoteric surfactants; peptides and derivatives thereof; protein hydrolyzates; swelling agents and penetrating agents; natural or synthetic thickeners for (a) oils; basifying agents, such as ethanol amine; acidifying agents; inorganic or organic fillers; antioxidants; preservatives; bactericides; suspending agents; sequestering agents; opacifying agents; dyes; organic or inorganic UV filters; vitamins or provitamins; moisturizing agents; self-tanning compounds; antiwrinkle active principles; fragrances; preserving agents, stabilizers; and mixtures thereof.

The amount of the additional ingredient(s) is not limited, but may be from 0.1 to 30% by weight relative to the total weight of the composition according to the present invention.

The composition of the present invention is in the form of an O/W emulsion having nano-sized oil droplets as the oil phase. The composition may be a cosmetic composition, preferably a cosmetic composition for a keratin substance, and more preferably a skin cosmetic composition. The composition can be in the form of a lotion, a milky lotion, a cream, a gel, a paste, a serum, or a foam, preferablyin the form of a gel, and more preferably a transparent or translucent gel.

The composition preferably exhibits a pH which is compatible with the skin and which generally ranges from 3 to 8 and preferably from 4.5 to 7.

The viscosity of the composition according to the present invention is not particularly limited. The viscosity can be measured at 25°C with viscosimeters or rheometers preferably with cone-plate or parallel-plate geometry. Preferably, the viscosity of the composition can range, for example, from 1 to 5000 Pa.s at 25°C and 21s⁻¹. In addition, the composition may possess a Newtonian nature.

The composition according to the present invention is preferably transparent or translucent. The transparency of the composition can be determined by measuring the turbidity with, for example, a trubidimeter (2100Q portable, Hach Company). Preferably, the composition has the turbidity more than 0, preferably more than 10, and less than 200, preferably less than 150.

The composition according to the present invention can be manufactured by first preparing a saturated oil phase with the active ingredients and then mixing the saturated oil phase with the water phase gently. The saturated oil phase with the active ingredients can be prepared by an excess amount of the active ingredient being dissolved in the oil and mixed vigorously at elevated temperature, and then cooled down to room temperature and maintained for several hours to days.

### [Cosmetic Process]

The composition according to the present invention may preferably be used as a cosmetic composition, in particular for skin-whitening or antiaging. In particular, the composition according to the present invention may be intended for application onto a keratin substance such as the skin, the scalp and/or the lips, preferably the skin. Thus, the composition according to the present invention can be used for a cosmetic process for the skin.

The cosmetic process or cosmetic use for a keratin substance such as skin, according to the present invention comprises, at least, the step of applying onto the keratin substance the composition according to the present invention.

The composition according to the present invention can be used in the topical skin care composition in the form of a lotion, a milky lotion, a cream, a gel, a paste, a serum, a foam, or a spray, preferably in the form of a gel or a spray, and more preferably a transparent or translucent gel.

### EXAMPLES

The present invention will be described in a more detailed manner by way of examples. However, these examples should not be construed as limiting the scope of the present invention.

### [Example 1 and Comparative Examples 1-3]

The following compositions according to Example 1 and Comparative Examples 1-3, shown in Table 1, were prepared as follows. First, an excess amount of 4-(tetrahydro-2H-pyran-4-yl)benzene-1,3-diol was mixed with isopropyl lauroyl sarcosinate vigorously at 80°C for 5 hours, cooled down to room temperature, and then maintained over 24 hours to obtain isopropyl lauroyl sarcosinate containing 4-(tetrahydro-2H-pyran-4-yl)benzene-1,3-diol (from CHEMEX) in a saturated concentration (a saturated oil). Then, the obtained saturated oil was mixed with a water phase comprising water, laureth-9 (from Nikko Chemicals Co.) and caprylyl/capryl glucoside (from SEPPIC) with or without dipropylene glycol gently at 80°C for 0.5 hours, cooled down to room temperature, and then maintained over 24 hours to yield O/W emulsion compositions according to Example 1 and Comparative Examples 1-3. The numerical values for the amounts of the ingredients are all based on "% by weight" as active raw materials.

**Table 1**

| | Ex. 1 | Comp. Ex. 1 | Comp. Ex. 2 | Comp. Ex. 3 |
|---|---|---|---|---|
| Laureth-9 | 4.8 | 4.8 | 4.8 | 4.8 |
| Caprylyl/Capryl Glucoside | 1 | 1 | 1 | 1 |
| Isopropyl Lauroyl Sarcosinate | 1.93 | 1.93 | 1.93 | 1.93 |
| 4-(tetrahydro-2H-pyran-4-yl)benzene-1,3-diol | 0.17 | 0.17 | 0.17 | 0.17 |
| Water | 92.1 | 82.1 | 72.1 | 62.1 |
| Dipropylene glycol | - | 10 | 20 | 30 |

### [Evaluation]

The compositions according to Example 1 and Comparative Examples 1-3 were evaluated as follows.

### (Droplet size)

Particle size analyzer (Vasco, Cordoun Technologies) was used to determine the droplet size of the oil phase of the O/W emulsion compositions according to Example 1 and Comparative Examples 1-3. The refractive index and viscosity of the solvent were 1.33 and 0.89 cp at 25 °C.

### (Saturation Degree of Active Ingredients)

The maximum solubility of 4-(tetrahydro-2H-pyran-4-yl)benzene-1,3-diol in isopropyl lauroyl sarcosinate was determined by measuring the amount of 4-(tetrahydro-2H-pyran-4-yl)benzene-1,3-diol dissolved in the saturated isopropyl lauroyl sarcosinate, which was obtained in the preparation process of the O/W compositions according to Example 1 and Comparative Examples 1-3, using a UV/VIS spectrophotometer type V-550 (JASCO, Japan) at 280 nm at room temperature and under atmospheric pressure. The concentration of 4-(tetrahydro-2H-pyran-4-yl)benzene-1,3-diol in the oil phase of each O/Wcompositions according to Example 1 and Comparative Examples 1-3 was determined by measuring the amount of 4-(tetrahydro-2H-pyran-4-yl)benzene-1,3-diol dissolved in the oil phase at room temperature using a UV/VIS spectrophotometer type V-550 (JASCO, Japan) at 280 nm at room temperature and under atmospheric pressure. In both cases, isopropyl lauroyl sarcosinate samples to be measured were prepared by separation with centrifuging at 14,000 rpm for 20 min.

The saturation degree (SD) of 4-(tetrahydro-2H-pyran-4-yl)benzene-1,3-diol was calculated with the following formula: SD = The concentration of 4-(tetrahydro-2H-pyran-4-yl)benzene-1,3-diol in the oil phase of the O/W emulsion / the maximum solubility of 4-(tetrahydro-2H-pyran-4-yl)benzene-1,3-diol in isopropyl lauroyl sarcosinate.

### (Penetration of Active Ingredient)

The penetration property of 4-(tetrahydro-2H-pyran-4-yl)benzene-1,3-diol in each composition was determined by using Strat-M membrane (registered trademark, sold by EMD Millipore Corporation, USA), which was used without further modification. The experimental procedure is described below in a detailed manner.

### Experimental Procudeure:

- Receptor fluid (RF) was prepared by mixing 0.25g Tween 80, 10g of phosphate buffered saline, and 89.75g of water in a beaker and stirring until it became isotropic. The obtained RF had a pH value of 7.4.
- A stirrer was put in a Frantz cell and the temperature of the cell was adjusted at 32°C by using a thermostat bath.
- 5 mL of the RF was introduced into the Frantz cell and wait until the temperature reaches at 32°C.
- Strat M membrane having 1.5cm² surface area was placed on the lower compartment of the cell. The lower and upper compartments of the cell were set up with a clamp tightly.
- 30 mg of the sample was placed on the Strat M membrane and spread evenly on the surface of the membrane.
- After a certain time (0.5h, 1h, 2h, 3h, 4h, and 5h), 200 µL of the RF was taken out with a syringe and the concentration of 4-(tetrahydro-2H-pyran-4-yl)benzene-1,3-diol past the membrane was determined by using a Ultra performance liquid Chromatography (UPLC) (Waters).
- 200 µL of the RF is placed into the Frantz cell.

### (Appearance)

The appearance of the compositions according to Example 1 and Comparative Examples 1-3 were determined by measuring their turbidities at room temperature by using a trubidimeter (2100Q portable, Hach Company). The meaning of the turbidity value (NTU) was defined as follows:

### NTU

| | |
|---|---|
| 1-50: | Transparent |
| 50-150: | Translucent |
| >200: | Turbid |

The results of these evaluations are shown in Table 2.

**Table 2**

| | Ex. 1 | Comp. Ex. 1 | Comp. Ex. 2 | Comp. Ex. 3 |
|---|---|---|---|---|
| Oil Droplet Size (nm) | 10 | 15 | 10 | 5 |
| Active SD | 1 | 0.95 | 0.92 | 0.5 |
| Active Penetration (%) | 74% | 70% | 63% | 48% |
| NTU | 20 | 19 | 20 | 10 |

The composition according to Example 1 was able to provide better penetration property of the active ingredient. Furthermore, the appearances of these compositions were translucent, which is preferable in use for skin cosmetic compositions.

On the other hand, the compositions according to Comparative Examples 1 to 3, which included more than 12% by weight of dipropylene glycol relative to the water, exhibited the lower penetration property of the active ingredient, even though they included the same amount of the active ingredient as the composition according to Example 1.

Thus, it is clear that the compositions according to the present invention can produce improved penetration property of the active ingredient and transparent aspect.

## Claims

1. A composition in the form of an O/W emulsion having an oil phase dispersed in an aqueous phase, said composition comprising:
(a) at least one oil;
(b) at least one active ingredient;
(c) water; and
(d) at least one surfactant selected from nonionic surfactants;
wherein
the diameter of the oil droplets of the oil phase is less than 30 nm, and
the aqueous phase comprises less than 5 % by weight of water soluble alcohol relative to the total amount of the aqueous phase,
the water soluble alcohol being an alcohol which can dissolve in an amount of 1 g or more in 100 mL water at room temperature and under atmospheric pressure,
the oil phase includes the active ingredient,
the amount of the active ingredient in the oil phase is ranging from 0.1 to 20% by weight, relative to the total weight of the oil phase, and
the active ingredient is selected from a group consisting of:
- resorcinol derivatives represented by formula (I): 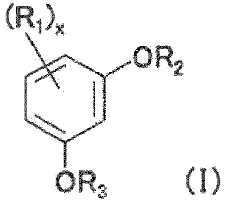 wherein
R₁ independently denotes -A-B where A represents a single bond, a C₁-C₆ alkylene group, a C₆-₁₂ arylene group, or a C₁₋₆ alkylene-C₆₋₁₂ arylene group, and B represents a halogen atom, -OH, -COH, -COOH, -CONH₂, -NH₂, a C₁-C₆ alkyl group, a C₁₋₆ alkoxy group, a C₁₋₆ acyl group, a carbocyclic group, preferably an aryl group, or heterocyclic group, preferably a non-aromatic heterocyclic group, each of which may be substituted with at least one substituent selected from the group consisting of a hydroxyl group, a carboxyl group, a C₁₋₆ alkyl group, a C₁₋₆ alkylene-OH, an amino group, -CONH₂, -CONH-C₁₋₆ alkyl group and a C₁₋₆ alkoxy group;
x is an integer of 1 to 4, preferably 1 to 3, more preferably 1 or 2 and even more preferably 1; and
R₂ and R₃ independently denote a hydrogen atom, a C₁₋₆ alkyl group, or a C₁₋₆ acyl group,
or a salt thereof,
- cinnamaldehyde derivatives selected from trans-ferulic acid, p-coumaric acid, and coniferylaldehyde, and
- a combination thereof,
wherein the amount of the water in the composition is from 60 to 99% by weight relative to the total weight of the composition.

2. The composition according to Claim 1, wherein the diameter of the oil phase is more than 1 nm, more preferably more than 2 nm, and even more preferably more than 5 nm.

3. The composition according to Claim 1 or 2, wherein the amount of the active ingredient in the oil phase is ranging from I to 15% by weight, and preferably from 3 to 10% by weight, relative to the total weight of the oil phase.

4. The composition according to any one of Claims 1 to 3, wherein the amount of the surfactant is from 0.1 to 20% by weight, preferably from 0.5 to 15% by weight, more preferably from 1 to 10% by weight, relative to the total weight of the composition.

5. The composition according to any one of Claims 1 to 4, wherein the amount of the oil is from 0.1 to 15% by weight, preferably from 0.5 to 10% by weight, and more preferably from 1 to 5% by weight, relative to the total weight of the composition.

6. The composition according to any one of Claims 1 to 5, wherein the amount of the water in the composition is from 70 to 98% by weight, and preferably 80 to 97% by weight, relative to the total weight of the composition.

7. The composition according to any one of Claims 1 to 6, wherein the oil is selected from a group consisting of ester oils having a molecular weight less than 600 g/mol such as isopropyl myristate, isopropyl palmitate, ethyl hexyl palmitate, isopropyl lauroyl sarcosinate, artificial triglycerides such as capryl caprylic triglycerides; hydrocarbon oil; and mineral oil such as paraffine oil, and mixtures thereof.

8. The composition according to Claim 7, wherein the active ingredient has a solubility in the (a) oil ranging from 0.01 to 50% by weight, preferably from 0.05 to 40% by weight, and more preferably from 0.1 to 30% by weight at room temperature and under atmospheric pressure.

9. The composition according to any one of Claims 1 to 8, wherein the surfactant is selected from a group consisting of ethers of a sugar and of C₈-C₂₄ fatty alcohols, such as caprylyl/capryl glucoside, polyoxyethylenated fatty alcohol containing from 6 to 12 oxyethylene units, such as Laureth-9, polyoxyalkylenated derivative of mono glyceryl ester of a fatty acid such as PEG-20 glyceryl triisostearate, polyglyceryl esters of a fatty acid, such as polyglyceryl-2 oleate, and mixtures thereof.

10. The composition according to any one of Claims 1 to 9, wherein an appearance of the composition is transparent or translucent.

11. The composition according to any one of Claims 1 to 10, wherein the active ingredient has a solubility of less than 0.5g, preferably less than 0.1g, in particular less than 0.01g relative to 100 g of water at room temperature and under atmospheric pressure.

12. A cosmetic process for a keratin substance such as skin, comprising the step of: applying onto the keratin substance the composition according to any one of Claims 1 to 11.

## Patentansprüche

1. Zusammensetzung in Form einer Ö/W-Emulsion mit einer in einer wässrigen Phase dispergierten Ölphase, wobei die Zusammensetzung Folgendes umfasst:
(a) mindestens ein Öl;
(b) mindestens einen Wirkstoff;
(c) Wasser; und
(d) mindestens ein Tensid, das aus nichtionischen Tensiden ausgewählt ist;
wobei
der Durchmesser der Öltröpfchen der Ölphase kleiner ist als 30 nm, und
die wässrige Phase weniger als 5 Gew.-% wasserlöslichen Alkohol umfasst, bezogen auf die Gesamtmenge der wässrigen Phase,
wobei es sich bei dem wasserlöslichen Alkohol um einen Alkohol handelt, der sich in einer Menge von 1 g oder mehr in 100 ml Wasser bei Raumtemperatur und unter Atmosphärendruck lösen kann,
die Ölphase den Wirkstoff enthält,
die Menge des Wirkstoffs in der Ölphase im Bereich von 0,1 bis 20 Gew.-% liegt, bezogen auf das Gesamtgewicht der Ölphase, und
der Wirkstoff ausgewählt ist aus einer Gruppe bestehend aus:
- Resorcinderivaten, die durch Formel (I) repräsentiert werden: 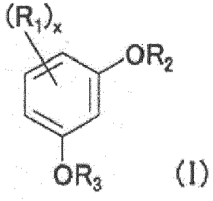 worin
R₁ unabhängig voneinander -A-B bezeichnet, wobei A eine Einfachbindung, eine C₁-C₆-Alkylengruppe, eine C₆₋₁₂-Arylengruppe oder eine C₁₋₆-Alkylen-C₆₋₁₂-Arylengruppe darstellt, und B ein Halogenatom, -OH, -COH, -COOH, -CONH₂, -NH₂, eine C₁-C₆-Alkylgruppe, eine C₁₋₆-Alkoxygruppe, eine C₁₋₆-Acylgruppe, eine carbocyclische Gruppe, vorzugsweise eine Arylgruppe oder eine heterocyclische Gruppe, vorzugsweise eine nichtaromatische heterocyclische Gruppe darstellt, die jeweils mit mindestens einem Substituenten substituiert sein kann, der aus der aus einer Hydroxylgruppe, einer Carboxylgruppe, einer C₁₋₆-Alkylgruppe, einer C₁₋₆-Alkylen-OH-Gruppe, einer Aminogruppe, -CONH₂-Gruppe, -CONH-C₁₋₆-Alkylgruppe und einer C₁₋₆-Alkoxygruppe bestehenden Gruppe ausgewählt ist;
x eine ganze Zahl von 1 bis 4, vorzugsweise 1 bis 3, mehr bevorzugt 1 oder 2 und noch mehr bevorzugt 1 ist; und
R₂ und R₃ unabhängig voneinander ein Wasserstoffatom, eine C₁₋₆-Alkylgruppe oder eine C₁₋₆-Acylgruppe oder ein Salz davon bezeichnen,
- Zimtaldehyd-Derivaten, die aus trans-Ferulasäure, p-Cumarsäure und Coniferylaldehyd ausgewählt sind, und
- einer Kombination davon,
wobei die Menge des Wassers in der Zusammensetzung von 60 bis 99 Gew.-% beträgt, bezogen auf das Gesamtgewicht der Zusammensetzung.

2. Zusammensetzung nach Anspruch 1, wobei der Durchmesser der Ölphase mehr als 1 nm, mehr bevorzugt mehr als 2 nm und noch mehr bevorzugt mehr als 5 nm beträgt.

3. Zusammensetzung nach Anspruch 1 oder 2, wobei die Menge des Wirkstoffs in der Ölphase im Bereich von 1 bis 15 Gew.-% und vorzugsweise im Bereich von 3 bis 10 Gew.-% liegt, bezogen auf das Gesamtgewicht der Ölphase.

4. Zusammensetzung nach einem der Ansprüche 1 bis 3, wobei die Menge des Tensids von 0,1 bis 20 Gew.-%, vorzugsweise von 0,5 bis 15 Gew.-%, mehr bevorzugt von 1 bis 10 Gew.-% beträgt, bezogen auf das Gesamtgewicht der Zusammensetzung.

5. Zusammensetzung nach einem der Ansprüche 1 bis 4, wobei die Menge des Öls von 0,1 bis 15 Gew.-%, vorzugsweise von 0,5 bis 10 Gew.-% und mehr bevorzugt von 1 bis 5 Gew.-% beträgt, bezogen auf das Gesamtgewicht der Zusammensetzung.

6. Zusammensetzung nach einem der Ansprüche 1 bis 5, wobei die Menge des Wassers in der Zusammensetzung von 70 bis 98 Gew.-% und vorzugsweise von 80 bis 97 Gew.-% beträgt, bezogen auf das Gesamtgewicht der Zusammensetzung.

7. Zusammensetzung nach einem der Ansprüche 1 bis 6, wobei das Öl aus einer aus Esterölen mit einem Molekulargewicht kleiner als 600 g/mol bestehenden Gruppe ausgewählt ist, wie zum Beispiel aus Isopropylmyristat, Isopropylpalmitat, Ethylhexylpalmitat, Isopropyllauroylsarcosinat, künstlichen Triglyceriden wie zum Beispiel Capryl-/Caprylsäuretriglyceriden; Kohlenwasserstofföl; und Mineralöl wie zum Beispiel Paraffinöl, und Mischungen davon.

8. Zusammensetzung nach Anspruch 7, wobei der Wirkstoff eine Löslichkeit in dem (a) Öl im Bereich von 0,01 bis 50 Gew.-%, vorzugsweise von 0,05 bis 40 Gew.-% und mehr bevorzugt von 0,1 bis 30 Gew.-% bei Raumtemperatur und unter Atmosphärendruck besitzt.

9. Zusammensetzung nach einem der Ansprüche 1 bis 8, wobei das Tensid aus einer aus Ethern eines Zuckers und von C₈-C₂₄-Fettalkoholen bestehenden Gruppe ausgewählt ist, wie zum Beispiel aus Caprylyl-/Caprylglucosid, polyoxyethyleniertem Fettalkohol, der von 6 bis 12 Oxyethyleneinheiten enthält, wie zum Beispiel Laureth-9, polyoxyalkyleniertes Derivat von Monoglycerylester einer Fettsäure, wie zum Beispiel PEG-20-Glyceryltriisostearat, Polyglycerylester einer Fettsäure, wie zum Beispiel Polyclyceryl-2-Oleat, und Mischungen davon.

10. Zusammensetzung nach einem der Ansprüche 1 bis 9, wobei ein Erscheinungsbild der Zusammensetzung durchsichtig oder durchscheinend ist.

11. Zusammensetzung nach einem der Ansprüche 1 bis 10, wobei der Wirkstoff eine Löslichkeit von weniger als 0,5 g, vorzugsweise weniger als 0,1 g, insbesondere weniger als 0,01 g bezogen auf 100 g Wasser bei Raumtemperatur und unter Atmosphärendruck besitzt.

12. Kosmetisches Verfahren für eine Keratinsubstanz wie zum Beispiel Haut, mit dem folgenden Schritt: Aufbringen der Zusammensetzung nach einem der Ansprüche 1 bis 11 auf die Keratinsubstanz.

## Revendications

1. Composition sous la forme d'une émulsion H/E ayant une phase huileuse dispersée dans une phase aqueuse, ladite composition comprenant :
(a) au moins une huile ;
(b) au moins un ingrédient actif ;
(c) de l'eau ; et
(d) au moins un surfactant sélectionné parmi des surfactants non ioniques ;
dans laquelle
le diamètre des gouttelettes d'huile de la phase huileuse est inférieur à 30 nm, et
la phase aqueuse comprend moins de 5 % en poids d'alcool hydrosoluble par rapport à la quantité totale de la phase aqueuse,
l'alcool hydrosoluble étant un alcool qui peut se dissoudre en une quantité de 1 g ou plus dans 100 ml d'eau à température ambiante et sous pression atmosphérique,
la phase huileuse inclut l'ingrédient actif,
la quantité de l'ingrédient actif dans la phase huileuse se trouvant dans la plage de 0,1 à 20 % en poids, par rapport au poids total de la phase huileuse, et l'ingrédient actif est sélectionné dans un groupe constitué par :
- des dérivés de résorcinol correspondant à la formule (1) : 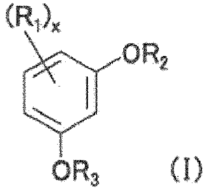 dans lesquels
R₁ représente indépendamment -A-B où A correspond à une liaison simple, un groupe alkylène en C₁-C₆, un groupe arylène en C₆-₁₂, ou un groupe (alkylène en C₁-₆) - (arylène en C₆-₁₂), et B correspond à un atome d'halogène, -OH, - COH, -COOH, -CONH₂, -NH₂, un groupe alkyle en C₁-C₆, un groupe alcoxy en C₁-₆, un groupe acyle en C₁-₆, un groupe carbocyclique, de préférence un groupe aryle, ou un groupe hétérocyclique, de préférence un groupe hétérocyclique non aromatique, chacun d'entre eux pouvant être substitué par au moins un substituant sélectionné dans le groupe constitué par un groupe hydroxyle, un groupe carboxyle, un groupe alkyle en C₁-₆, un groupe (alkylène en C₁-₆)-OH, un groupe amino, -CONH₂, un groupe -CONH-(alkyle en C₁-₆) et un groupe alcoxy en C₁-₆ ;
x est un nombre entier de 1 à 4, de préférence de 1 à 3, de manière davantage préférée de 1 ou 2 et de manière encore davantage préférée de 1 ; et
R₂ et R₃ représentent indépendamment un atome d'hydrogène, un groupe alkyle en C₁-₆ ou un groupe acyle en C₁-₆,
ou un sel de celui-ci,
- des dérivés de cinnamaldéhyde sélectionnés parmi l'acide trans-férulique, l'acide p-coumarique, et le coniférylaldéhyde, et
- une combinaison de ceux-ci,
dans laquelle la quantité de l'eau dans la composition est de 60 à 99 % en poids par rapport au poids total de la composition.

2. Composition selon la revendication 1, dans laquelle le diamètre de la phase huileuse est supérieur à 1 nm, de manière davantage préférée supérieur à 2 nm, et de manière encore davantage préférée supérieur à 5 nm.

3. Composition selon la revendication 1 ou 2, dans laquelle la quantité d'ingrédient actif dans la phase huileuse se trouve dans la plage de 1 à 15 % en poids, et de préférence de 3 à 10 % en poids, par rapport au poids total de la phase huileuse.

4. Composition selon l'une quelconque des revendications 1 à 3, dans laquelle la quantité du surfactant est de 0,1 à 20 % en poids, de préférence de 0,5 à 15 % en poids, de manière davantage préférée de 1 à 10 % en poids, par rapport au poids total de la composition.

5. Composition selon l'une quelconque des revendications 1 à 4, dans laquelle la quantité de l'huile est de 0,1 à 15 % en poids, de préférence de 0,5 à 10 % en poids, et de manière davantage préférée de 1 à 5 % en poids, par rapport au poids total de la composition.

6. Composition selon l'une quelconque des revendications 1 à 5, dans laquelle la quantité de l'eau dans la composition est de 70 à 98 % en poids, et de préférence de 80 à 97 % en poids, par rapport au poids total de la composition.

7. Composition selon l'une quelconque des revendications 1 à 6, dans laquelle l'huile est sélectionnée dans un groupe constitué par des huiles d'ester ayant une masse moléculaire inférieure à 600 g/mol par exemple le myristate d'isopropyle, le palmitate d'isopropyle, le palmitate d'éthyl hexyle, le lauroyl sarcosinate d'isopropyle, les triglycérides artificiels tels que les triglycérides capriques capryliques ; une huile hydrocarbonée ; et une huile minérale telle que l'huile de paraffine, et des mélanges de ceux-ci.

8. Composition selon la revendication 7, dans laquelle l'ingrédient actif a une solubilité dans (a) de l'huile dans la plage de 0,01 à 50 % en poids, de préférence de 0,05 à 40 % en poids, et de manière davantage préférée de 0,1 à 30 % en poids à température ambiante et sous pression atmosphérique.

9. Composition selon l'une quelconque des revendications 1 à 8, dans laquelle le surfactant est sélectionné dans un groupe constitué par des éthers de sucre et des alcools gras en C₈-C₂₄, par exemple un glycoside caprylylique/caprylique, un alcool gras polyoxyéthyléné contenant de 6 à 12 unités oxyéthylène, par exemple le laureth-9, un dérivé polyoxyalkyléné de monoglycéryl ester d'un acide gras tel que le triisostéarate de glycéryle de PEG-20, des polyglycéryl ester d'un acide gras, tels que l'oléate de polyglycéryle-2, et des mélanges de ceux-ci.

10. Composition selon l'une quelconque des revendications 1 à 9, dans laquelle un aspect de la composition est transparent ou translucide.

11. Composition selon l'une quelconque des revendications 1 à 10, dans laquelle l'ingrédient actif a une solubilité inférieure à 0,5 g, de préférence inférieure à 0,1 g, en particulier inférieure à 0,01 g pour 100 g d'eau à température ambiante et sous pression atmosphérique.

12. Procédé cosmétique pour une substance kératinique telle que la peau, comprenant l'étape suivante : application sur la substance kératinique de la composition selon l'une quelconque des revendications 1 à 11.
